(19) Europäisches Patentamt
European Patent Office
Office européen des brevets

(11) EP 3 194 977 B1

(12) **EUROPEAN PATENT SPECIFICATION**

(45) Date of publication and mention of the grant of the patent:
**02.10.2019 Bulletin 2019/40**

(21) Application number: **15753233.4**

(22) Date of filing: **21.07.2015**

(51) Int Cl.:
***G01N 33/68*** *(2006.01)*

(86) International application number:
**PCT/US2015/041353**

(87) International publication number:
**WO 2016/014546 (28.01.2016 Gazette 2016/04)**

# (54) DETERMINATION OF PROTEIN AGGREGATION FROM THE CONCENTRATION DEPENDENCE OF DELTA G

BESTIMMUNG DER PROTEINAGGREGATION DER KONZENTRATIONSABHÄNGIGKEIT VON DELTA G

DÉTERMINATION DE L'AGRÉGATION DE PROTÉINES À PARTIR DE LA DÉPENDANCE À LA CONCENTRATION DE DELTA G

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.07.2014 US 201462026984 P**
**07.08.2014 US 201462034504 P**

(43) Date of publication of application:
**26.07.2017 Bulletin 2017/30**

(73) Proprietor: **Unchained Labs**
**Pleasanton, California 94566 (US)**

(72) Inventors:
- **FREIRE, Ernesto**
**North Bethesda MD 20852 (US)**
- **SCHON, Arne**
**Baltimore, Maryland 21211 (US)**
- **BROWN, Richard**
**East Falmouth, Massachusetts 02536 (US)**

(74) Representative: **Cooley (UK) LLP**
**Dashwood**
**69 Old Broad Street**
**London EC2M 1QS (GB)**

(56) References cited:
**WO-A1-2005/103686 WO-A2-2012/027361**

- **FRIDA OJALA ET AL: "Prediction of IgG1 aggregation in solution", BIOTECHNOLOGY JOURNAL, vol. 9, no. 6, 15 June 2014 (2014-06-15), pages 800-804, XP055223981, DE ISSN: 1860-6768, DOI: 10.1002/biot.201400018**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 194 977 B1

## Description

### CROSS-REFERENCE TO RELATED APPLICATIONS

This application claims priority to, and the benefit of, U.S. Provisional Application No. 62/026,984, filed July 21, 2014 and U.S. Provisional Application No. 62/034,504, filed August 07, 2014.

### BACKGROUND OF THE INVENTION

Protein aggregation is a major issue affecting the long-term stability of protein preparations, especially biologics. Often, aggregates originate from the presence of small amounts of denatured or partially denatured protein in the formulation. These protein conformations are prone to aggregate, for example, when a protein's hydrophobic core becomes exposed to a solvent. The aggregation of denatured protein gradually shifts the protein equilibrium towards increasing amounts of denatured and ultimately aggregated denatured protein. In other situations, the native state itself may show a tendency to aggregate, a predisposition that is exacerbated in antibody drug conjugates (ADC's) since the attached small molecule drugs are often highly hydrophobic.

Thus, there exists a need for new methods and systems for recognizing and characterizing protein aggregation processes. Such methods and systems will provide enormous benefits in (1) the identification and selection of protein formulations that minimize aggregation and extend long-term stability and (2) the identification of protein variants with the lowest tendency to aggregate. The present application addresses such a need.

### SUMMARY OF THE INVENTION

The present invention relates to methods and systems for determining and characterizing protein aggregation processes at the earliest possible time and use of such new methods and systems for (1) the identification and selection of protein formulations that minimize aggregation and extend long-term stability and (2) the identification of protein variants with the lowest tendency to aggregate.

A first aspect of the present invention relates to a method for the amount or fraction of protein aggregation in a solution based upon said solution's protein concentration. The method including steps of (1) providing a plurality of first solutions comprising increasing concentrations of the protein; (2) measuring an observable property for each solution in the plurality of first solutions; (3) determining $\Delta G$ for the conformational stability of the protein at each concentration in the plurality of first solutions based on the observable property; (4) creating a correlation between $\Delta G$ and each concentration of the protein; and (5) using the correlation to determine the amount and/or fraction of the protein that is aggregated and/or the amount and/or fraction of the protein that is denatured.

As used herein "denaturation" is a process in which proteins lose the quaternary structure, tertiary structure and secondary structure which are present in their native state, by application of some external stress or reagent such as a strong acid or base, a concentrated inorganic salt, an organic solvent, radiation or heat. Commonly used chemical denaturants include urea and guanidine hydrochloride. Protein denaturation in living cells may result in disruption of cellular activity and possibly cell death. *In vivo* or *in vitro,* denatured proteins can exhibit a wide range of characteristics, from conformational change and loss of solubility to aggregation due to the exposure of hydrophobic groups. In this process, exposed hydrophobic portions of the unfolded protein may interact with the exposed hydrophobic patches of other unfolded proteins, spontaneously leading to protein aggregation.

As used herein "protein aggregation" is a phenomenon in which proteins aggregate (*i.e*., accumulate and clump together) intracellularly or extracellularly (including *in vitro*). When proteins in pharmaceutical acceptable formulations aggregate, the effective dose of the formulation is reduced and they may cause unwanted immunological responses. Proteins may aggregate in their native states or denatured states.

$\Delta G$, the thermodynamic parameter that determines the structural stability and/or conformational stability of the protein is measured by evaluating the resistance of a protein to increasing amounts of a physical or chemical denaturing agent. A physical agent is temperature or pressure. In this case, denaturation is achieved by progressively increasing the temperature or pressure of the protein solution. A chemical agent is a denaturant molecule like urea or guanidine hydrochloride. In this case, protein denaturation is achieved by progressively increasing the concentration of denaturant. The denaturation of the protein is accompanied by changes in some observable properties of the protein. Analyses of these changes are used to evaluate $\Delta G$. In embodiments, a plurality of solutions will contain no chemical denaturant or a concentration of the chemical denaturant which is insufficient to produce denatured proteins at a detectable level.

In some embodiments, an observable property is fluorescence. When a protein denatures, certain measurable characteristics of the protein also change. One such characteristic is the fluorescence of the protein. Fluorescence may be inherent to the protein, *e.g*., *via* amino acids that naturally fluoresce, *e.g*., tryptophan, tyrosine, and phenylalanine, or may be extrinsic to the protein, *e.g*., *via* a probe comprising a fluorescent label. Other methods known in the art for detecting the denatured state of a protein and/or that allow determination of ΔG may be used. There are many physical observable properties and their associated instrumentation, in addition to fluorescence spectroscopy, that are sensitive to the degree of denaturation of a protein. These observable properties may be measured by various techniques including, but not limited to uv/vis spectroscopy, circular dichroism, nuclear magnetic resonance (NMR), infrared spectroscopy (IR), and differential scanning calorimetry, among others.

In some embodiments, the method may further comprise the steps of (1) providing a plurality of at least second solutions comprising increasing concentrations of the protein; (2) measuring an observable property for each solution in the plurality of at least second solutions; (3) determining ΔG for the conformational stability of the protein at each concentration in the plurality of at least second solutions based on the observable property; (4) creating a correlation between ΔG and each concentration of the protein for each plurality of at least second solutions; and (5) using the correlation to determine the amount and/or fraction of the protein that is aggregated and/or the amount and/or fraction of the protein that is denatured in the plurality of at least second solutions.

In embodiments, the method includes a step of comparing the ΔG for the conformational stability of the protein at each concentration in the plurality of first solutions with the ΔG for the conformational stability of the protein at each concentration in the plurality of at least second solutions.

In some embodiments, a plurality of at least second solutions comprises a plurality of second solutions. In some embodiments, a plurality of at least second solutions comprises a plurality of second solutions and a plurality of third solutions. In some embodiments, a plurality of at least second solutions comprises a plurality of second solutions, a plurality of third solutions, and a plurality of fourth solutions. In some embodiments, a plurality of at least second solutions comprises a plurality of second solutions, a plurality of third solutions, a plurality of fourth solutions, and a plurality of fifth solutions. In some embodiments, a plurality of at least second solutions comprises a plurality of second solutions, a plurality of third solutions, a plurality of fourth solutions, a plurality of fifth solutions, and more pluralities of solutions (*e.g.*, a plurality of sixth solutions, a plurality of seventh solutions, a plurality of eighth solutions, a plurality of ninth solutions, a plurality of tenth solutions, a plurality of twentieth solutions, a plurality of fortieth solutions, a plurality of sixtieth solutions, a plurality of eightieth solutions, a plurality of hundredth solutions, and any number of pluralities in between). Embodiments include more than one hundred pluralities of solutions.

One plurality of solutions may differ from another plurality of solutions in one or more *(e.g.,* one, two, three, four, five, six, seven, and eight) conditions or factors that may be adjusted for making the solutions. In some embodiments, the conditions or factors are selected from the group consisting of buffer composition, buffer strength, pH, ionic strength, excipient composition, excipient concentration, chemical denaturant composition, and chemical denaturant concentration. In some embodiments, one plurality of solutions differs from another plurality of solutions in only one condition or factor. In some embodiments, one plurality of solutions differs from another plurality of solutions only in buffer composition, buffer strength, pH, ionic strength, excipient composition, excipient concentration, chemical denaturant composition, or chemical denaturant concentration. In some embodiments, each of the plurality of at least second solutions differs from the plurality of first solutions only in buffer composition, buffer strength, pH, ionic strength, excipient composition, excipient concentration, chemical denaturant composition, or chemical denaturant concentration. In some embodiments, each of the plurality of at least second solutions differs from the plurality of first solutions only in chemical denaturant concentration. In some embodiments, each of the plurality of at least second solutions comprises higher concentrations of chemical denaturant than the plurality of first solutions. In some embodiments, the plurality of first solutions comprises no chemical denaturant or an amount of chemical denaturant that produces an undetectable amount or undetectable fraction of denatured protein.

Each solution in a plurality of solutions may differ from another solution in the plurality of solutions in one or more (*e.g.*, one, two, three, four, five, six, seven, and eight) conditions or factors that may be adjusted for making the solutions (*e.g.*, the conditions or factors described herein). In some embodiments, each solution in a plurality of solutions differs in no more than three conditions (*e.g.*, no condition, one condition, two conditions, and three conditions) selected from the group consisting of buffer composition, buffer strength, pH, ionic strength, excipient composition, excipient concentration, chemical denaturant composition, and chemical denaturant concentration.

In a non-limiting example, each solution in a plurality of first solutions may have a pH of 5.7, comprise 0.9% NaCl, and 3M Urea and each solution in a plurality of at least second solutions have a pH of 6.7, comprise 0.9% NaCl, and 3M Urea, with the only difference being the pH between the plurality of first solutions and the plurality of at least second solutions. In another non-limiting example, each solution in a plurality of first solutions may have a pH of 5.7, comprise 1.2% NaCl, and 3M Urea and each solution in a plurality of at least second solutions have a pH of 6.7, comprise 0.9% NaCl, and 4M urea, with the difference being the pH and the concentration of the denaturant (*i.e.*, urea) between the plurality of first solutions and the plurality of at least second solutions. **Figure 16** illustrates exemplary solutions useful in the present invention.

As used herein "increasing concentration" means that the solutions in a plurality of solutions of the present application (*e.g.*, a plurality of first solutions, a plurality of second solutions, a plurality of third solutions, *etc*.) have a range of concentrations of a component (*e.g.*, the protein) that increases from the lowest concentration to the highest concentration. For example, the solutions in a plurality of solutions comprise concentrations of the protein that are 0.1 μg/ml, 0.2 μg/ml, 0.5 μg/ml, 1 μg/ml, 5 μg/ml, 50 μg/ml, 100 μg/ml, 200 μg/ml, 500 μg/ml, and 1 mg/ml, respectively.

Two or more pluralities of solutions may comprise solutions of the same concentrations of the protein. In some embodiments, the plurality of first solutions and the plurality of at least second solutions comprise solutions of the same concentrations of the protein. In some embodiments, the plurality of first solutions and the plurality of second solutions comprise solutions of the same concentrations of the protein. In a non-limiting example, a plurality of first solutions includes ten solutions having concentrations of a proteins that are 0.1 μg/ml, 0.2 μg/ml, 0.5 μg/ml, 1 μg/ml, 5 μg/ml, 50 μg/ml, 100 μg/ml, 200 μg/ml, 500 μg/ml, and 1 mg/ml, respectively, and a plurality of at least second solutions includes ten solutions having concentrations of said protein that correspond to the concentrations in the plurality of first solutions, *i.e.*, 0.1 μg/ml, 0.2 μg/ml, 0.5 μg/ml, 1 μg/ml, 5 μg/ml, 50 μg/ml, 100 μg/ml, 200 μg/ml, 500 μg/ml, and 1 mg/ml, respectively.

In some embodiments, the method may further comprise the step of creating a correlation between ΔG for the conformational stability of the protein and one or more (*e.g.*, one, two, three, four, five, six, seven, and eight) conditions or factors that may be adjusted for making the solutions (*e.g.*, the conditions or factors described herein) for each solution of the plurality of the first solutions and each solution of the plurality of at least second solutions.

In embodiments, the method may further comprise providing a plurality of solutions (*i.e.*, "chemical denaturant solutions") having the same concentration of protein (and/or other composition components) and increasing concentrations of a chemical denaturant (*e.g.*, urea and guanidine hydrochloride). The correlation of increasing concentrations of the chemical denaturant on ΔG, on the amount and/or fraction of the protein that is denatured, and/or on the amount and/or fraction of the protein that is aggregated is determined (*e.g.*, by measuring an observable property that is capable of monitoring protein denaturation). In a non-limiting example, the plurality of solutions includes ten solutions having an identical concentration of the protein and concentrations of urea between 0 and 10 M, *e.g.*, 0 M, 1 M, 2 M, 3.0 M, 3.25 M, 3.5 M, 3.75 M, 4 M, 6 M, and 8 M.

In some embodiments, the method may further include a step of comparing ΔG values for each concentration of the protein to determine whether the protein aggregates in its native state or aggregates in a denatured state.

In some embodiments, the method may further include a step of determining the amount and/or fraction of the protein that is denatured for each different concentration. In some embodiments, the method may further include a step of determining the amount and/or fraction of the protein that is aggregated for each different concentration. In some embodiments, the method may further include a step of determining the amount and/or fraction of the protein that is denatured and the amount and/or fraction of the protein that is aggregated for each different concentration.

In some embodiments, the method may further comprise a step of comparing the amount and/or fraction of the protein that is aggregated and/or the amount and/or fraction of the protein that is denatured in the plurality of first solutions with the amount and/or fraction of the protein that is aggregated or the amount and/or fraction of the protein that is denatured in the plurality of at least second solutions.

In some embodiments, the method may further comprise a step of creating a correlation between the amount and/or fraction of the protein that is aggregated and one or more (*e.g.*, one, two, three, four, five, six, seven, and eight) conditions or factors that may be adjusted for making the solutions (*e.g.*, the conditions or factors described herein) for each solution of the plurality of the first solutions and each solution of the plurality of at least second solutions. In some embodiments, the method may further comprise a step of creating a correlation between the amount and/or fraction of the protein that is denatured and one or more (*e.g.*, one, two, three, four, five, six, seven, and eight) conditions or factors that may be adjusted for making the solutions (*e.g.*, the conditions or factors described herein) for each solution of the plurality of the first solutions and each solution of the plurality of at least second solutions. In some embodiments, the method may further comprise a step of creating a correlation between the amount and/or fraction of the protein that is aggregated and the amount and/or fraction of the protein that is denatured and one or more (*e.g.*, one, two, three, four, five, six, seven, and eight) conditions or factors that may be adjusted for making the solutions (*e.g.*, the conditions or factors described herein) for each solution of the plurality of the first solutions and each solution of the plurality of at least second solutions.

In some embodiments, the method may further comprise using the correlation between the amount and/or fraction of the protein that is aggregated and/or the amount and/or fraction of the protein that is denatured and one or more (*e.g.*, one, two, three, four, five, six, seven, and eight) conditions or factors that may be adjusted for making the solutions (*e.g.*, the conditions or factors described herein) to determine the one or more (*e.g.*, one, two, three, four, five, six, seven, and eight) conditions or factors that minimize the amount and/or fraction of the aggregated and/or denatured protein.

In some embodiments, the method may detect 2 ng/ml or less denatured protein (*e.g.*, 0.5 ng/ml to 1.5 ng/ml, 0.75 ng/ml to 1.25 ng/ml, less than 1 ng/ml, and every concentration in between) and/or determine 2 ng/ml or less aggregated protein (*e.g.*, 0.5 ng/ml to 1.5 ng/ml, 0.75 ng/ml to 1.25 ng/ml, less than 1 ng/ml, and every concentration in between).

ΔG may be determined within 24 hours (*e.g.*, less than 20 hours, less than 18 hours, less than 15 hours, less than 12 hours, less than 10 hours, less than 8 hours, less than 6 hours, less than 4 hours, less than 3 hours, less than 2 hours, less than 1 hour, and every time in between).

In some embodiments, the amount and/or fraction of the protein that is denatured and the amount and/or fraction of the protein that is aggregated is may be determined within 24 hours (*e.g.*, less than 20 hours, less than 18 hours, less than 15 hours, less than 12 hours, less than 10 hours, less than 8 hours, less than 6 hours, less than 4 hours, less than 3 hours, less than 2 hours, less than 1 hour, and every time in between).

A plurality of solutions may include at least 3 to 20 solutions (*e.g.*, 3, 4, 5, 6, 7, 8, 9, 10, 11, 12, 13, 14, 15, 16, 17, 18, 19, and 20 solutions) with each solution comprising the protein at a different concentration. Each solution in a plurality of solutions may include less than 200 mg/ml of a protein (*e.g.*, less than 100 mg, less than 50 mg/ml, less than 20 mg/ml, less than 10 mg/ml, less than 5 mg/ml, less than 2 mg/ml, less than 1 mg/ml, and any concentration in between). In some embodiments, a solution comprises 0.1 μg/ml to less than 2 mg/ml of the protein (*e.g.*, less than 0.2 μg/ml, less than 0.5 μg/ml, less than 1 μg/ml, less than 2 μg/ml, less than 3 μg/ml, less than 4 μg/ml, less than 5 μg/ml, less than 10 μg/ml, less than 20 μg/ml, less than 50 μg/ml, less than 100 μg/ml, less than 200 μg/ml, less than 500 μg/ml, less than 1 mg/ml, and any concentration in between).

One or more pluralities of solutions may be subject to conditions that promote protein denaturation and/or aggregation. In some embodiments, the denaturation is induced by a chemical denaturant (*e.g.*, urea and guanidine hydrochloride). In some embodiments, the denaturation is induced by heat. In some embodiments, the denaturation is induced by a chemical denaturant in combination with heat.

In embodiments, the herein described methods are performed *in vitro*, *ex vivo*, or *in vivo.* In embodiments, the herein described methods are performed *in vitro*, *i.e.*, in a designed and prepared, non-naturally-occurring solution. In embodiments, each component/factor and amount of each component/factor in a solution is preselected and combined to produce the solution.

In some embodiments, the method may further include a step of determining a concentration of the protein in which ΔG's dependence on the protein's concentration is minimized in a solution. In embodiments, the method further includes a step of identifying a protein concentration that maximizes ΔG and minimizes ΔG's protein-concentration dependence.

In embodiments, the method further includes a step of using the correlation between ΔG and each concentration of the protein in the plurality of first solutions to determine the amount or fraction of said protein that is denatured and the fraction of said denatured protein that is aggregated in said plurality of first solutions. In embodiments, the method further includes a step of using the correlation between ΔG and each concentration of the protein in the plurality of at least second solutions to determine the amount or fraction of said protein that is denatured and the fraction of said denatured protein that is aggregated in said plurality of at least solutions. Embodiments further include comparing the amount or fraction of the protein that is aggregated or the amount or fraction of the protein that is denatured in the plurality of first solutions with the amount or fraction of the protein that is aggregated or the amount or fraction of the protein that is denatured in the plurality of at least second solutions.

In embodiments, the method further includes a step of selecting one or more conditions that further maximizes ΔG and minimizes ΔG's protein-concentration dependence selected from the group consisting of buffer composition, buffer strength, pH, ionic strength, excipient composition, and excipient concentration.

The above aspect further provides a method for preparing a protein formulation (e.g., a pharmaceutically acceptable protein formulation) which has a protein concentration in which ΔG's dependence on the protein's concentration is minimized in a solution. In embodiments, the formulation has a protein concentration that maximizes ΔG for the conformational stability of the protein and minimizes ΔG's protein-concentration dependence. In embodiments, the formulation has a protein concentration and includes one or more conditions (selected from buffer composition, buffer strength, pH, ionic strength, excipient composition, and excipient concentration) that maximizes ΔG for the conformational stability of the protein and minimizes ΔG's protein-concentration dependence. In embodiments, selecting the protein formulation comprising a step of selecting one or more conditions and a concentration of the protein which minimizes the amount or fraction of the protein that is aggregated and/or minimizes the amount or fraction of the protein that is denatured.

An aspect of the present invention is a protein formulation (*e.g.*, pharmaceutically acceptable protein formulation) having one or more conditions (selected from buffer composition, buffer strength, pH, ionic strength, excipient composition, and excipient concentration) and a concentration of said protein (which minimizes protein concentration dependence ΔG and/or minimizes the amount or fraction of said protein that is aggregated) as determined by a method described herein. In embodiments, the formulation has a protein concentration and includes one or more conditions that maximizes ΔG for the conformational stability of the protein and minimizes ΔG's protein-concentration dependence. The protein formulation may include one or more conditions and a concentration of the protein which minimizes the amount or fraction of the protein that is aggregated and/or minimizes the amount or fraction of the protein that is denatured.

As used herein a pharmaceutically acceptable protein formulation is a liquid (*e.g.*, an aqueous) preparation of the protein which is both stable (shelf and when *in vivo*) and acceptable to a patient, *e.g.*, an animal, preferably a mammal, and more preferably a human. The pharmaceutically acceptable protein formulation combines the protein with a variety of compounds to ensure a stable active medication following storage. These include, but are not limited to, solubilizers, stabilizers, buffers, tonicity modifiers, bulking agents, viscosity enhancers/reducers, surfactants, chelating agents, ligands and adjuvants.

Another aspect of the present invention relates to a method for determining a stable variant of a protein among a plurality of variants of the protein comprising steps of: (1) providing a plurality of first solutions comprising increasing concentrations of a first protein variant; (2) measuring an observable property for each solution in the plurality of first solutions; (3) determining ΔG for the conformational stability of the first protein variant at each concentration in the plurality of first solutions based on the observable property; (4) creating a correlation between ΔG and each concentration of the first protein variant; and (5) using the correlation to determine the amount or fraction of the protein that is aggregated (6) providing a plurality of at least second solutions comprising increasing concentrations of at least a second protein variant; (7) measuring an observable property for each solution in the plurality of at least second solutions; (8) determining ΔG for the conformational stability of the at least second protein variant at each concentration in the plurality of at least second solutions based on the observable property; (9) creating a correlation between ΔG and each concentration of the at least second protein variant, and (10) using the correlation to determine the amount or fraction of the protein that is aggregated, wherein the protein variant having a maximized ΔG and a minimized ΔG's protein-concentration dependence and/or has the lesser amount or fraction of aggregated protein is determined to be a stable protein variant. In embodiments, each solution in the plurality of first solutions differs in one or more conditions selected from the group consisting of buffer composition, buffer strength, pH, ionic strength, excipient composition, excipient concentration, chemical denaturant composition, and chemical denaturant concentration and each solution in said plurality of at least second solutions differs in one or more conditions selected from the group consisting of buffer composition, buffer strength, pH, ionic strength, excipient composition, excipient concentration, chemical denaturant composition, and chemical denaturant concentration. In embodiments, each solution in the plurality of first solutions differs in at least chemical denaturant concentration and each solution in the plurality of at least second solutions differs in at least chemical denaturant concentration.

Protein variants are proteins that differ in one or more amino acids and such difference may affect the proteins' stability (*i.e.*, tendency to denature and/or aggregate). A non-limiting example of protein variants includes monoclonal antibodies obtained from different hybridoma clones. Protein variants may also be proteins that differ in glycosylation and proteins that have been modified by the attachment (*e.g.*, covalent attachment) of small molecules; antibody drug conjugates are examples of such modified proteins.

As used in this Specification and the appended claims, the singular forms "a," "an" and "the" include plural referents unless the context clearly dictates otherwise.

Unless specifically stated or obvious from context, as used herein, the term "or" is understood to be inclusive and covers both "or" and "and", unless the context clearly dictates otherwise.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

Any aspect or embodiment described herein can be combined with any other aspect or embodiment as disclosed herein. While the disclosure has been described in conjunction with the detailed description thereof, the foregoing description is intended to illustrate and not limit the scope of the disclosure, which is defined by the scope of the appended claims. Other aspects, advantages, and modifications are within the scope of the following claims.

Unless defined otherwise, all technical and scientific terms used herein have the same meaning as commonly understood by one of ordinary skill in the art to which the invention pertains. Although other probes, compositions, methods, and kits similar, or equivalent, to those described herein can be used in the practice of the present invention, the preferred materials and methods are described herein. It is to be understood that the terminology used herein is for the purpose of describing particular embodiments only, and is not intended to be limiting.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. All other published references, documents, manuscripts and scientific literature cited herein are hereby incorporated by reference.

From the foregoing description, it will be apparent that variations and modifications may be made to the invention described herein to adopt it to various usages and conditions. Such embodiments are also within the scope of the following claims.

## BRIEF DESCRIPTION OF THE DRAWINGS

The patent or application file contains at least one drawing executed in color. Copies of this patent or patent application publication with color drawings will be provided by the Office upon request and payment of the necessary fee.

**Figure 1** shows a protein's conformational equilibrium between the native (red) and denatured (green) (unfolded or partially unfolded) states. Under some conditions the denatured state (and also the native state) may exhibit a tendency to self-associate or aggregate.

**Figure 2** illustrates the dependence of ΔG on protein concentration which provides a quantitative assessment of a protein's tendency to aggregate in their native state or in their denatured state.

**Figure 3** illustrates factors which affect ΔG.

**Figure 4:** (A) includes a graph the relationship between ΔG and HIV-1 Protease concentration. HIV-1 Protease's ΔG increases with increased protein concentration.
(B) includes a graph showing the relationship between ΔG and α-Chymotrypsin concentration. α-Chymotrypsin's ΔG decreases with increased protein concentration.

**Figure 5** includes a graph showing the relationship between ΔG and Trastuzumab concentration. Data from two formulations are shown: Trastuzumab in a 5% dextrose solution (in blue) and Trastuzumab in 0.9% NaCl solution (in green). Trastuzumab's ΔG increases with increased protein concentration in the 5% dextrose solution.

**Figures 6** includes graphs showing the relationship between ΔG and Cetuximab concentration (A), between the fraction of denatured Cetuximab and Cetuximab concentration (B), between the fraction of aggregated Cetuximab and Cetuximab concentration (C), and between the fraction of the aggregated, denatured Cetuximab and Cetuximab concentration (D). For each panel, the solution used for the data shown in blue is identical to the solution for the data shown in red, which was supplemented with 300 mM L-Arginine. (A) shows that Cetuximab's ΔG decreases with increased protein concentration.

**Figure 7** includes graphs showing data from a study in which a monoclonal antibody was tested in two formulations. The graphs show the relationship between ΔG and the monoclonal antibody's concentration (A), between the fraction of denatured monoclonal antibody and the monoclonal antibody's concentration (B), between the fraction of the aggregated monoclonal antibody and the monoclonal antibody's concentration (C), and between the fraction of the aggregated, denatured monoclonal antibody and the monoclonal antibody's concentration (D). (A) shows that the monoclonal antibody's ΔG decreases with increased protein concentration.

**Figure 8** shows the relationship between ΔG and the protein concentration of monoclonal antibody-A (top left), between the fraction of denatured monoclonal antibody-A and monoclonal antibody-A's concentration (top right), between the fraction of aggregated monoclonal antibody-A and monoclonal antibody-A's concentration (bottom left), and between the fraction of the aggregated, denatured monoclonal antibody-A and monoclonal antibody-A's concentration (bottom right) for a pH 6.25 formulation of monoclonal antibody-A.

**Figure 9** shows the relationship between ΔG and the protein concentration of monoclonal antibody-A (top left), between the fraction of denatured monoclonal antibody-A and monoclonal antibody-A's concentration (top right), between the fraction of aggregated monoclonal antibody-A and monoclonal antibody-A's concentration (bottom left), and between the fraction of the aggregated, denatured monoclonal antibody-A and monoclonal antibody-A's concentration (bottom right) for a pH 6.75 formulation of monoclonal antibody-A.

**Figure 10** shows the relationship between ΔG and the protein concentration of monoclonal antibody-A (top left), between the fraction of denatured monoclonal antibody-A and monoclonal antibody-A's concentration (top right), between the fraction of aggregated monoclonal antibody-A and monoclonal antibody-A's concentration (bottom left), and between the fraction of the aggregated, denatured monoclonal antibody-A and monoclonal antibody-A's concentration (bottom right) for a pH 7.75 formulation of monoclonal antibody-A.

**Figure 11** is a graph overlaying the ΔG for the three formulations of Figures 8 to 10. The graph shows that monoclonal antibody-A's ΔG decreases with increased protein concentration.

**Figure 12** is a graph overlaying the fraction of denatured monoclonal antibody-A for the three formulations of Figures 8 to 10.

**Figure 13** is a graph overlaying the fraction of aggregated monoclonal antibody-A for the three formulations of Figures 8 to 10.

**Figure 14** is a graph overlaying the fraction of aggregated, denatured monoclonal antibody-A for the three formulations of Figures 8 to 10.

**Figure 15** is a plot showing changes in $\Delta G$ based on a formulation's pH and protein concentration. An ideal protein formulation has maximal $\Delta G$ versus solvents, constant $\Delta G$ versus time, and constant $\Delta G$ versus protein concentration. Superimposed upon the plot is a $\Delta G$ versus protein concentration graph for a formulation having a specific pH; identified on the superimposed graph (left arrow) is a protein concentration having a maximal $\Delta G$, but significant protein concentration dependence, and identified on the superimposed graph (right arrow) is a protein concentration having minimal protein concentration dependence. The later protein concentration is considered a "multiparametric optimal condition".

**Figure 16** is a diagram showing non-limiting examples of solutions and pluralities of solutions that may be included in the present invention.

## DETAILED DESCRIPTION OF THE INVENTION

The present invention relates to methods and systems for recognizing and characterizing protein aggregation processes at the earliest possible time and use of such new methods and systems for (1) the identification and selection of protein formulations that minimize aggregation and extend long-term stability and (2) the identification of protein variants with the lowest tendency to aggregate.

The present invention provides a method that allows quantitative determination of the amount of aggregated protein rapidly (e.g., within 24 hours). This novel approach is based upon the concentration dependence of the Gibbs energy of stability ($\Delta G$) of a protein. Measuring $\Delta G$ at different protein concentrations provides the necessary information to calculate the amount of protein that is aggregated in either the denatured or native states. In general, if $\Delta G$ decreases with protein concentration, aggregation in the denatured state occurs, whereas if $\Delta G$ increases with protein concentration, aggregation in the native state occurs. If $\Delta G$ does not change with protein concentration, there is no tendency to aggregate. *See,* **Figure 2.** Since $\Delta G$ measures the structural stability of a protein, it follows that the goal in identifying the best formulation or the best protein variant that maximizes $\Delta G$ and simultaneously minimizes $\Delta G$'s concentration dependence. *See,* **Figure 3.** Disclosed herein are quantitative analyses of $\Delta G$'s concentration dependence and methods for estimating aggregation soon after a protein solution is prepared. Early knowledge of structural stability and aggregation tendencies provides critical information to define specific measures aimed at minimizing protein aggregation and prolong the long-term stability of the protein.

The identification of conditions that maximize the structural stability of the native state of proteins and prevent aggregation or other undesirable processes is essential for the development of protein therapeutics, protein formulations, process development conditions, quality control as well as basic protein sciences.

In the field of protein therapeutics there are two stages at which identifying aggregation tendencies is critical: (1) the selection of the best protein variant for development (commonly referred to as developability assessment) and (2) the identification of formulation conditions that ensure the longest possible shelf life. Long-term stability of biologics is usually hampered by the appearance and slow growth of denatured protein aggregates that slowly reduce the concentration of native protein. This process not only diminishes the amount of biologically active protein, thus lowering therapeutic potency, but also can trigger undesirable adverse effects, including immune responses. Identifying the best protein variant or the best formulation requires the ability to measure both the concentration of denatured protein and the fraction of that protein that is aggregated in a timely fashion. Using this information, the protein variant or the formulation with the optimal stability and aggregation attributes can be rapidly determined.

Here, two different types of proteins are considered: proteins that tend to aggregate in their denatured state and proteins that tend to aggregate in their native state.

### *I. Denatured-State Aggregation*

Direct measurement of the population of denatured protein immediately after a formulation or protein solution is prepared is extremely difficult. The population of denatured protein in a fresh formulation is exceedingly small (usually less than 0.01% of the total protein) which escape detection by conventional techniques. Despite this fact, denatured protein aggregation is a major obstacle to long-term stability as it slowly grows and eventually destroys the viability of a protein stock. Commonly used approaches to assess denatured state aggregation, like accelerated stability or forced degradation assays, essentially aim at accelerating the accumulation of denatured and aggregated population to measurable levels. These approaches take months and are not entirely reliable as some of the stresses used to accelerate aggregation do not mimic real life situations. An alternative method to determine the population of denatured protein immediately after the protein solution is prepared relies on the evaluation of the structural equilibrium between native and denatured conformations. Determination of the Gibbs energy of stability ($\Delta G^o$) allows calculation of the amount of denatured protein in a solution to exceedingly low levels (<<0.001% of the total protein). Furthermore, since aggregation and conformational equilibrium are thermodynamically linked, we will show that the protein concentration dependence of $\Delta G^o$ provides critical information to evaluate the degree of protein aggregation in the formulation.

**Figure 1** illustrates the basic thermodynamic equilibrium proteins in solution undergo. The native state (red) exists in equilibrium with the denatured state (green) which has a tendency to aggregate in an eventually irreversible manner. The population or fraction of denatured protein ($F_{denat}$) is equal to the total concentration of denatured protein (non-aggregated plus aggregated) divided by the total protein concentration:

$$F_{denat} = \frac{[D]+j[D_j]}{[N]+[D]+j[D_j]} \qquad (1)$$

where j is the average degree of oligomerization of the denatured aggregates.

In the absence of aggregation, **Equation 1** reduces to the well-known equation:

$$F_{denat} = \frac{[D]}{[N]+[D]} = \frac{K}{1+K} = \frac{e^{-\frac{\Delta G^o}{RT}}}{1+e^{-\frac{\Delta G^o}{RT}}} \qquad (2)$$

which, at any constant temperature, is only a function of the Gibbs energy of stability ($\Delta G^o$). K is the equilibrium constant $(K = \frac{[D]}{[N]})$ which is also defined in terms of $\Delta G^o$. **Equation** 2 can be easily generalized to the situation in which a protein exhibits multiple transitions as each transition will be described by an equation similar to **Equation 1 or 2.**

When protein aggregation or self-association is absent, the fraction of denatured protein is independent of protein concentration as it only depends on $\Delta G^o$ (right hand side of **Equation 2)**.

The situation is different if aggregation occurs, since aggregation is a concentration-dependent phenomenon. Without being bound by theory, denatured-protein aggregation will increase the value of K while native aggregation will decrease its value; these changes affect the magnitude of $\Delta G^o$. An experimental test that evaluates the presence or absence of aggregation or self-association is measurement of $\Delta G^o$ at different protein concentrations. If $\Delta G^o$ is independent of concentration, no aggregation is present. If $\Delta G^o$ decreases with concentration, denatured-protein aggregation is present, whereas if $\Delta G^o$ increases with concentration native state-protein aggregation is present.

One method for experimentally determining $\Delta G^o$ is differential scanning calorimetry (DSC). This method involves thermal denaturation of proteins. Since thermal denaturation of many proteins, especially monoclonal antibodies which comprise the vast majority of biologics, is an irreversible process, DSC cannot be used to measure such protein's thermodynamic stability. Thus, use of DSC is limited to those proteins that exhibit reversible temperature denaturation.

A well-established method for experimentally determining $\Delta G^o$ consists of measuring the denaturation of the protein with a chemical denaturant, e.g., urea and guanidinium hydrochloride. The Gibbs energy has been experimentally shown to follow a simple linear dependence with the chemical denaturant's concentration:

$$\Delta G = \Delta G^o - m[denaturant] \qquad (3)$$

where $\Delta G^o$ is the Gibbs energy of stability of the protein at zero chemical denaturant concentration; *i.e.*, the stability of the protein at the experimental temperature and solvent conditions absent the chemical denaturant. m is the m-value.

Chemical denaturation of antibodies is usually reversible under conditions at which temperature denaturation is not, thus allowing $\Delta G^o$ determination. Chemical denaturation can be performed at different protein concentrations, thus allowing direct measurement of the concentration dependence of $\Delta G^o$.

***Concentration Dependence of the Gibbs Energy***

If the denatured state has a tendency to aggregate **(Figure 1)**, the aggregation process can be described in terms of a phenomenological aggregation constant $K_j = \frac{[D_j]}{[D]^j}$ in which j is the average degree of oligomerization of the aggregates. Here, the apparent or observed equilibrium constant, $K_{app}$, is defined as the ratio of the total concentration of denatured protein (non-aggregated plus aggregated) to the concentration of native protein:

$$K_{app} = \frac{[D]+j[D_j]}{[N]} = K(1 + jK_j[D]^{j-1}) \qquad (4)$$

Correspondingly, the apparent Gibbs energy becomes:

$$\Delta G_{app} = \Delta G_O^o - RTln(1 + jK_j[D]^{j-1}) \qquad (5)$$

In **Equation 5**, $\Delta G_O^o$ is equal to the value of the Gibbs energy extrapolated to zero protein concentration (in practice, a concentration low enough at which aggregation is not present). **Equation 5** indicates that denatured state aggregation will cause the measured Gibbs energy, $\Delta G_{app}$, to decrease as a function of protein concentration and that the magnitude of the decrease is proportional to the aggregation constant and the average size of the aggregates. If a protein aggregates in the native state, the concentration dependence of $\Delta G_{app}$ will be described by a similar equation to **Equation 5** except that the minus sign will be replaced by a plus sign. In this case, the measured Gibbs energy, $\Delta G_{app}$, will increase with protein concentration.

**Figure 4B** shows the experimental concentration dependence of $\Delta G$ and $\alpha$-Chymotrypsin concentration. $\alpha$-Chymotrypsin's $\Delta G$ decreases with increased protein concentration; thus, $\alpha$-Chymotrypsin is a protein that aggregates in its denatured state. Similarly, In **Figure 6A**, the experimental concentration dependence of $\Delta G_{app}$ for Cetuximab is shown. In this case $\Delta G_{app}$ decreases with protein concentration as expected for a protein that aggregates in the denatured state.

***The Population of Aggregated Protein***

The fraction of protein that is aggregated is calculated according to the following standard equations:

$$F_{agg} = \frac{j[D_j]}{([N]+[D]+j[D_j])} = \frac{jK_j[D]^{j-1}}{(\frac{1}{K}+1+jK_j[D]^{j-1})} \qquad (6)$$

From **Equation 5**, it is apparent that the term $jK_j[D]^{j-1}$ can be expressed solely in terms of the measured $\Delta G$ if its concentration dependence is known:

$$F_{agg} = \frac{(e^{-\frac{\Delta\Delta G}{RT}}-1)}{(e^{\frac{\Delta G_O^o}{RT}}+1+\left(e^{-\frac{\Delta\Delta G}{RT}}-1\right))} \qquad (7)$$

where $\Delta\Delta G = \Delta G_{app} - \Delta G_O^o$. **Equation 7** allows estimation of the fraction of aggregated protein without a need to know the free denatured concentration ([D]), the aggregation constant, and the average degree of aggregation. In a similar fashion, the fraction of denatured protein, $F_{denat}$, can be expressed as:

$$F_{denat} = \frac{e^{-\frac{\Delta\Delta G}{RT}}}{(e^{\frac{\Delta G_O^o}{RT}}+1+\left(e^{-\frac{\Delta\Delta G}{RT}}-1\right))} \qquad (8)$$

In a typical formulation situation, the vast majority of protein is in the native conformation and therefore the fraction of denatured protein is exceedingly small. For a typical protein in which ΔG is on the order of 8 kcal/mol, the denatured fraction is on the order of $10^{-6}$. From the point of view of long-term stability, a critical quantity is the fraction of denatured protein that is aggregated ($F_{agg\ of\ Fdenat}$). Denatured protein that aggregates, even though initially the amount is very small, will act as a sink that over an extended period of time will shift the equilibrium towards increasing amounts of denatured and aggregated protein. This progressive aggregation process will eventually make the solution useless or therapeutically unviable. The fraction of denatured protein that is aggregated is given by the equation:

$$F_{agg\ of\ F_{denat}} = \frac{e^{-\frac{\Delta\Delta G}{RT}}-1}{(1+\left(e^{-\frac{\Delta\Delta G}{RT}}-1\right))} \qquad (9)$$

**Figure 6B** to **D** shows the three fractional populations that can be obtained from the analysis of the data in **Figure 6A** for Cetuximab. Similarly, **Figure 7A** shows the experimental concentration dependence of $\Delta G_{app}$ for a monoclonal antibody and **Figure 7B** to **D** shows the three fractional populations that can be obtained from the analysis of the data in **Figure 7A.** Also, **Figure 8** to **Figure 11** show experimental concentration dependence of $\Delta G_{app}$ for monoclonal Antibody-A and **Figure 8** to **Figure 10** and **Figure 12** to **Figure 14** show the three fractional populations that can be obtained from the analysis of the experimental data.

As shown in the figures, the amount of denatured protein and aggregated protein is exceedingly small, as expected in a freshly prepared solution. The denatured fraction amounts to about $10^{-6}$. These low amounts are impossible to measure with conventional techniques (*e.g.*, size exclusion chromatography and light scattering) but can be accurately calculated from $\Delta G_{app}$. From the point of view of long-term stability, the important observation is that most of the denatured protein aggregates. Even at the relatively low concentration of 0.18 mg/mL, more than 95% of the denatured protein is aggregated. Denatured protein with a high tendency to aggregate will eventually deplete the population of native and active protein and result in poor long-term stability.

A successful formulation strategy is one that minimizes the amount of denatured protein and simultaneously minimizes the fraction of aggregated denatured protein. From a thermodynamic point of view that strategy means one that is geared to simultaneously maximize $\Delta G_{app}$ and minimize its protein concentration dependence. *See* **Figure 15**

Following the above-described methods, for a protein that aggregates in a denatured state, an ideal protein formulation having maximal ΔG versus solvents, constant ΔG versus time, and ΔG versus protein concentration (*i.e.*,. minimal protein concentration dependence) can be determined. Likewise, the above-described methods, for a protein that aggregates in a denatured state, an protein variant having maximal ΔG versus solvents, constant ΔG versus time, and ΔG versus protein concentration (*i.e.*,. minimal protein concentration dependence) relative to other variants of the protein can be determined.

### *II. Native-State Aggregation*

If the native state has a tendency to aggregate (**Figure 1, left side**), the aggregation process can be described in terms of a phenomenological aggregation constant, $K_{N,i}$, similar to the one described above for denatured state aggregation $K_{N,i} = \frac{[N_i]}{[N]^i}$ in which i is the average degree of oligomerization of the native state aggregates. In this case, the apparent or observed equilibrium constant, $K_{app}$, is defined as the ratio of the total concentration of denatured protein to the concentration of native protein (non-aggregated plus aggregated):

$$K_{app} = \frac{[D]}{[N]+i[N_i]} = \frac{K}{(1+iK_{N,i}[N]^{i-1})} \qquad (10)$$

correspondingly, the apparent Gibbs energy becomes:

$$\Delta G_{app} = \Delta G_o^o + RTln(1 + iK_{N,i}[N]^{i-1}) \qquad (11)$$

In **Equation 11,** $\Delta G_o^o$ is equal to the value of the Gibbs energy extrapolated to zero protein concentration (in practice, a concentration low enough at which aggregation is not present). **Equation 11** indicates that native state aggregation will cause the measured Gibbs energy, $\Delta G_{app}$, to increase as a function of protein concentration and that the magnitude of the increase is proportional to the aggregation constant and the average size of the aggregates. This equation is similar to **Equation 5** except that the minus sign is replaced by a plus sign.

***The Population of Native-State Aggregated Protein***

The fraction of protein that is aggregated is calculated according to the following standard equations:

$$F_{agg} = \frac{i[N_i]}{([N]+i[N_i]+[D])} = \frac{iK_{N,i}[N]^{i-1}}{(1+iK_{N,i}[N]^{i-1}+K)} \qquad (12)$$

As in the case of denatured aggregation, the term $iK_{N,i}\,[N]^{i-1}$ can be expressed solely in terms of the measured $\Delta G$ if its concentration dependence is known:

$$F_{agg} = \frac{(e^{\frac{\Delta\Delta G}{RT}}-1)}{(1+e^{-\frac{\Delta G_O^o}{RT}}+\left(e^{\frac{\Delta\Delta G}{RT}}-1\right))} \qquad (13)$$

where $\Delta\Delta G = \Delta G_{app} - \Delta G_O^o$. **Equation 13** is analogous to **Equation 7** and it allows estimation of the fraction of native state aggregated protein without a need to know the aggregation constant, and the average degree of aggregation. In a similar fashion, the fraction of denatured protein, $F_{denat}$, can be expressed as:

$$F_{denat} = \frac{e^{-\frac{\Delta G_O^o}{RT}}}{(1+e^{-\frac{\Delta G_O^o}{RT}}+\left(e^{\frac{\Delta\Delta G}{RT}}-1\right))} \qquad (8)$$

In a typical formulation situation, the vast majority of protein is in the native conformation and therefore the fraction of denatured protein is exceedingly small. For a typical protein in which $\Delta G$ is on the order of 8 kcal/mol, the denatured fraction is on the order of $10^{-6}$. From the point of view of long-term stability, a critical quantity is the fraction of denatured protein that is aggregated ($F_{agg\ of\ Fdenat}$).

**Figure 4A** shows the experimental concentration dependence of $\Delta G$ for HIV-1 Protease concentration; HIV-1 Protease's $\Delta G$ increases with increased protein concentration; thus, HIV-1 Protease is a protein that is a dimer in its native state. **Figure 5** shows the experimental concentration dependence of $\Delta G_{app}$ for trastuzumab, which is known to readily aggregate in the presence of 5% dextrose. In this case, $\Delta G_{app}$ increases with protein concentration, as predicted for a protein that aggregates in the native state.

A successful formulation strategy is one that minimizes the amount of denatured protein and simultaneously minimizes the fraction of aggregated denatured protein. From a thermodynamic point of view that strategy means one that is geared to simultaneously maximize $\Delta G_{app}$ and minimize its protein concentration dependence.

Following the above-described methods, for a protein that aggregates in a native state, an ideal protein formulation having maximal $\Delta G$ versus solvents, constant $\Delta G$ versus time, and $\Delta G$ versus protein concentration (*i.e*.,. minimal protein concentration dependence) can be determined. Likewise, the above-described methods, for a protein that aggregates in a native state, an protein variant having maximal $\Delta G$ versus solvents, constant $\Delta G$ versus time, and $\Delta G$ versus protein concentration (*i.e*.,. minimal protein concentration dependence) relative to other variants of the protein can be determined.

A system of the present invention may include an apparatus with a controller having a processing unit and a storage element. The storage element may be RAM, DRAM, ROM, Flash ROM, EEROM, magnetic media, or any other medium suitable to hold computer readable data and instructions. The instructions may be those necessary to execute calculations of, at least, any equation described herein. The processing unit may be a dedicated microcontroller, a personal computer or any other suitable computing device. In addition, the apparatus has a pump or siphon system, which allows it to extract liquid from a variety of wells in exact quantities and mix these liquids together, preferably in another well. The apparatus also has a means to measure and record the fluorescence of formulations, such as by using a cannula to draw liquid into a commercially available fluorescence detector. The apparatus also includes one or more actuators which can move cannulas from one position to another, so as to draw fluid from a first well and expel the fluid into a second well. These cannulas can be used to prepare the formulations needed to create a denaturation graph, and to prepare the formulation of protein in buffer.

Methods, systems, and apparatuses useful in the present invention have been described previously, e.g., in U.S. 8,859,295, U.S. 8,609,040, U.S. 9,029,163, and U.S. 2012/0045846.

**REFERENCES**

U.B. Ericsson, B.M. Hallberg, G.T. DeTitta, N. Dekker, P. Nordlund, Thermofuor-based high-throughput stability optimization of proteins for structural studies, Anal. Biochem, 357 (2006) 289-298.

M. Vedadi, F.H. Niesen, A. Allali-Hassani, O.Y. Fedorov, P.J. Finerty Jr, G.A. Wasney, R. Yeung, C. Arrowsmith, L.J. Ball, H. Berglund, R. Hui, B.D. Marsden, P. Nordlund, M. Sundstrom, J. Weigelt, A.M. Edwards, Chemical screening methods to identify ligands that promote protein stability, protein crystallization and structure determination, Proc. Natl. Acad. Sci. (USA), 103 (2006) 15835-15840.

M.A.H. Capelle, R. Gurny, T. Arvinte, High throughput screening of protein formulation stability: Practical considerations, Eurpean J. Pharmaceutics and Biopharmaceutics, 65 (2007) 131-148.

G.A. Senisterra, P.J. Finerty Jr., High throughput methods of assessing protein stability and aggregation, Mol. BioSyst., 5 (2009) 217-223.

E.Y. Chi, S. Krishnan, T.W. Randolph, J.F. Carpenter, Physical Stability of Proteins in Aqueous Solution: Mechanism and Driving Forces in Nonnative Protein Aggregation, Pharmaceutical Research, 20 (2003) 1325-1336.

O. Boudker, M.J. Todd, E. Freire, The structural stability of the co-chaperonin GroES, Journal of molecular biology, 272 (1997) 770-779.

M.J. Todd, N. Semo, E. Freire, The structural stability of the HIV-1 protease, Journal of molecular biology, 283 (1998) 475-488.

R.F. Greene, Jr., C.N. Pace, Urea and guanidine hydrochloride denaturation of ribonuclease, lysozyme, alpha-chymotrypsin, and beta-lactoglobulin, J. Biol. Chem., 249 (1974) 5388-5393.

C.N. Pace, D.V. Laurents, R.E. Erickson, Urea denaturation of barnase: pH dependence and characterization of the unfolded state, Biochemistry, 31 (1992) 2728-2734.

M.M. Santoro, D.W. Bolen, Unfolding free energy changes determined by the linear extrapolation method. 1. Unfolding of phenylmethanesulfonyl.alpha.chymotrypsin using different denaturants, Biochemistry, 27 (1988) 8063-8068.

D.W. Bolen, M.M. Santoro, Unfolding free energy changes determined by the linear extrapolation method. 2. Incorporation of dGn-u values in a thermodynamic cycle, Biochemistry, 27 (1988) 8069-8074.

J.K. Myers, C.N. Pace, J.M. Scholtz, Denaturant m values and heat capacity changes: Relation to changes in accessible surface area of protein unfolding, Prot. Science, 4 (1995) 2138-2148.

C.N. Pace, Determination and analysis of urea and guanidine hydrochloride denaturation curves, Methods Enzymol, 131 (1986) 266-280.

R.M. Ionescu, J. Vlasak, C. Price, M. Kirchmeier, Contribution of variable domains to the stability of humanized IgG1 monoclonal antibodies, Journal of Pharmaceutical Sciences, 97 (2008) 1414-1426.

E. Freire, A. Schon, B.M. Hutchins, R.K. Brown, Chemical denaturation as a tool in the formulation optimization of biologics, Drug Discovery Today, 18 (2013) 1007-1013.

B. Demeule, C. Palais, G. Machaidze, R. Gurny, T. Arvinte, New methods allowing the detection of protein aggregates:

A case study on trastuzumab, mAbs, 1 (2009) 142-150.

T. Arakawa, D. Eljima, K. Tsumoto, N. Obeyama, Y. Tanaka, Y. Kita, E.N. Timasheff, Suppression of protein interactions by arginine: a proposed mechanism of the arginine effects, Biophys Chem, 127 (2007) 1-8.

P. Kheddo, M. Tracka, J. Armer, R.J. Dearman, S. Uddin, C.F. van der Walle, A.P. Golovanov, The effect of arginine glutamate on the stability of monoclonal antibodies in solution, International Journal of Pharmaceutics, 473 (2014) 126-133.

M. Fukuda, D. Kameoka, T. Torizawa, M. Saitoh, M. Yasutake, Y. Imaeda, A. Kioga, A. Mizutani, Thermodynamic and fluorescence analyses to determine mechanisms of IgG1 stabilization and destabilization by arginine, Pharmaceutical Research, 31 (2014) 992-1001.

A. Schon, R.K. Brown, B. Hutchins, E. Freire, Ligand binding analysis and screening by chemical denaturation shift, Analytical Biochem, 443 (2013) 52-57.

## Claims

1. A method for determining the amount or fraction of protein aggregation in a solution based upon said solution's protein concentration, comprising:

   providing a plurality of first solutions comprising increasing concentrations of a protein;
   measuring an observable property for each solution in said plurality of first solutions;
   determining ΔG for the conformational stability of said protein at each concentration in said plurality of first solutions based on said observable property;
   creating a correlation between ΔG and each concentration of said protein; and
   using said correlation to determine the amount or fraction of said protein that is aggregated.

2. The method of claim 1, wherein each solution in said plurality of first solutions differs in no condition or in no more than three conditions selected from the group consisting of buffer composition, buffer strength, pH, ionic strength, excipient composition, excipient concentration, chemical denaturant composition, and chemical denaturant concentration.

3. The method of claim 1, further comprising:

   providing a plurality of at least second solutions comprising increasing concentrations of said protein;
   measuring an observable property for each solution in said plurality of at least second solutions;
   determining ΔG for the conformational stability of said protein at each concentration in said plurality of at least second solutions based on said observable property;
   creating a correlation between ΔG and each concentration of said protein in said plurality of at least second solutions; and
   using said correlation to determine the amount or fraction of said protein that is aggregated in said plurality of at least second solutions;
   wherein said plurality of at least second solutions differs from said plurality of first solutions in one or more conditions selected from the group consisting of buffer composition, buffer strength, pH, ionic strength, excipient composition, excipient concentration, chemical denaturant composition, and chemical denaturant concentration.

4. The method of claim 3, wherein each solution in said plurality of at least second solutions differs in no condition or in no more than three conditions selected from the group consisting of buffer composition, buffer strength, pH, ionic strength, excipient composition, excipient concentration, chemical denaturant composition, and chemical denaturant concentration.

5. The method of claim 3, wherein said plurality of at least second solutions comprises solutions having the same concentrations of said protein as said plurality of first solutions.

6. The method of claim 3, further comprising creating, for each solution of the plurality of the first solutions and each solution of the plurality of at least second solutions, a correlation between ΔG and a condition selected from the group consisting of buffer composition, buffer strength, pH, ionic strength, excipient composition, and excipient concentration.

**7.** The method of claim 3, further comprising creating a correlation between the amount or fraction of said protein that is aggregated and a condition selected from the group consisting of buffer composition, buffer strength, pH, ionic strength, excipient composition, and excipient concentration.

**8.** The method of claim 3, further comprising comparing the amount or fraction of said protein that is aggregated in said plurality of first solutions with the amount or fraction of said protein that is aggregated in said plurality of at least second solutions.

**9.** The method of claim 1, wherein 1 ng/ml or less denatured protein or 1 ng/ml or less aggregated protein is detected or determined.

**10.** The method of claim 1, wherein the amount or fraction of the protein that is aggregated is determined within 24 hours.

**11.** The method of claim 1, wherein each solution in said plurality of first solutions comprises 0.1 μg/ml to 2 mg/ml of said protein.

**12.** The method of claim 3, wherein each solution in said plurality of at least second solutions comprises 0.1 μg/ml to 2 mg/ml of said protein.

**13.** The method of claim 1, wherein said observable property is fluorescence.

**14.** The method of claim 3, wherein said plurality of at least second solutions differs from said plurality of first solutions in at least chemical denaturant concentration.

**15.** The method of claim 1, further comprising a step of identifying a protein concentration that maximizes ΔG and minimizes ΔG's protein-concentration dependence.

**16.** The method of claim 1, further comprising a step of using said correlation to determine the amount or fraction of said protein that is denatured and the fraction of said denatured protein that is aggregated in said plurality of first solutions.

**17.** The method of claim 16, further comprising steps of
determining the amount or fraction of said protein that is denatured and the fraction of said denatured protein that is aggregated in said plurality of at least second solutions; and
comparing the amount or fraction of said protein that is aggregated or the amount or fraction of said protein that is denatured in said plurality of first solutions with the amount or fraction of said protein that is aggregated or the amount or fraction of said protein that is denatured in said plurality of at least second solutions.

**Patentansprüche**

**1.** Verfahren zur Bestimmung der Menge oder des Anteils einer Proteinaggregation in einer Lösung auf der Basis der Proteinkonzentration der Lösung, umfassend:

Bereitstellen von mehreren ersten Lösungen, die ansteigende Konzentrationen eines Proteins umfassen;
Messen einer beobachtbaren Eigenschaft für jede Lösung in den mehreren ersten Lösungen;
Bestimmen von ΔG für die Konformationsstabilität des Proteins bei jeder Konzentration in den mehreren ersten Lösungen auf der Basis der beobachtbaren Eigenschaft;
Erzeugen einer Korrelation zwischen ΔG und jeder Konzentration des Proteins; und
Verwenden der Korrelation zum Bestimmen der Menge oder der Fraktion des Proteins, das aggregiert ist.

**2.** Verfahren nach Anspruch 1, wobei jede Lösung in den mehreren ersten Lösungen sich in Bezug auf keinen Zustand oder in Bezug auf nicht mehr als drei Zustände unterscheidet, die aus der Gruppe bestehend aus Pufferzusammensetzung, Pufferstärke, pH-Wert, Ionenstärke, Hilfsstoffzusammensetzung, Hilfsstoffkonzentration, Zusammensetzung des chemischen Denaturierungsmittels und Konzentration des chemischen Denaturierungsmittels ausgewählt sind.

3. Verfahren nach Anspruch 1, weiterhin umfassend:

Bereitstellen von mehreren mindestens zweiten Lösungen, die ansteigende Konzentrationen des Proteins umfassen;
Messen einer beobachtbaren Eigenschaft für jede Lösung in den mehreren mindestens zweiten Lösungen;
Bestimmen von ΔG für die Konformationsstabilität des Proteins bei jeder Konzentration in den mehreren mindestens zweiten Lösungen auf der Basis der beobachtbaren Eigenschaft;
Erzeugen einer Korrelation zwischen ΔG und jeder Konzentration des Proteins in den mehreren mindestens zweiten Lösungen; und
Verwenden der Korrelation zum Bestimmen der Menge oder der Fraktion des Proteins, das aggregiert ist, in den mehreren mindestens zweiten Lösungen;
wobei die mehreren mindestens zweiten Lösungen sich von den mehreren ersten Lösungen in Bezug auf einen oder mehrere Zustände unterscheiden, die aus der Gruppe bestehend aus Pufferzusammensetzung, Pufferstärke, pH-Wert, Ionenstärke, Hilfsstoffzusammensetzung, Hilfsstoffkonzentration, Zusammensetzung des chemischen Denaturierungsmittels und Konzentration des chemischen Denaturierungsmittels ausgewählt sind.

4. Verfahren nach Anspruch 3, wobei jede Lösung in den mehreren mindestens zweiten Lösungen sich in Bezug auf keinen Zustand oder in Bezug auf nicht mehr als drei Zustände unterscheidet, die aus der Gruppe bestehend aus Pufferzusammensetzung, Pufferstärke, pH-Wert, Ionenstärke, Hilfsstoffzusammensetzung, Hilfsstoffkonzentration, Zusammensetzung des chemischen Denaturierungsmittels und Konzentration des chemischen Denaturierungsmittels ausgewählt sind.

5. Verfahren nach Anspruch 3, wobei die mehreren mindestens zweiten Lösungen Lösungen mit denselben Konzentrationen des Proteins wie die mehreren ersten Lösungen umfassen.

6. Verfahren nach Anspruch 3, weiterhin umfassend ein Erzeugen einer Korrelation zwischen ΔG und einem Zustand, der aus der Gruppe bestehend aus Pufferzusammensetzung, Pufferstärke, pH-Wert, Ionenstärke, Hilfsstoffzusammensetzung und Hilfsstoffkonzentration ausgewählt ist, für jede Lösung der mehreren der ersten Lösungen und jede Lösung der mehreren mindestens zweiten Lösungen.

7. Verfahren nach Anspruch 3, weiterhin umfassend ein Erzeugen einer Korrelation zwischen der Menge oder der Fraktion des Proteins, das aggregiert ist, und einem Zustand, der aus der Gruppe bestehend aus Pufferzusammensetzung, Pufferstärke, pH-Wert, Ionenstärke, Hilfsstoffzusammensetzung und Hilfsstoffkonzentration ausgewählt ist.

8. Verfahren nach Anspruch 3, weiterhin umfassend ein Vergleichen der Menge oder der Fraktion des Proteins, das aggregiert ist, in den mehreren ersten Lösungen mit der Menge oder der Fraktion des Proteins, das aggregiert ist, in den mehreren mindestens zweiten Lösungen.

9. Verfahren nach Anspruch 1, wobei 1 ng/ml oder weniger denaturiertes Protein oder 1 ng/ml oder weniger aggregiertes Protein erfasst oder bestimmt wird.

10. Verfahren nach Anspruch 1, wobei die Menge oder die Fraktion des Proteins, das aggregiert ist, innerhalb von 24 Stunden bestimmt wird.

11. Verfahren nach Anspruch 1, wobei jede Lösung in den mehreren ersten Lösungen 0,1 μg/ml bis 2 mg/ml des Proteins umfasst.

12. Verfahren nach Anspruch 3, wobei jede Lösung in den mehreren mindestens zweiten Lösungen 0,1 μg/ml bis 2 mg/ml des Proteins umfasst.

13. Verfahren nach Anspruch 1, wobei die beobachtbare Eigenschaft Fluoreszenz ist.

14. Verfahren nach Anspruch 3, wobei die mehreren mindestens zweiten Lösungen sich von den mehreren ersten Lösungen in Bezug auf mindestens die Konzentration des chemischen Denaturierungsmittels unterscheiden.

15. Verfahren nach Anspruch 1, weiterhin umfassend einen Schritt eines Identifizierens einer Proteinkonzentration, die ΔG maximiert und die Proteinkonzentrationsabhängigkeit von ΔG minimiert.

**16.** Verfahren nach Anspruch 1, weiterhin umfassend einen Schritt eines Verwendens der Korrelation zum Bestimmen der Menge oder der Fraktion des Proteins, das denaturiert ist, und der Fraktion des denaturierten Proteins, das aggregiert ist, in den mehreren ersten Lösungen.

**17.** Verfahren nach Anspruch 16, weiterhin umfassend die folgenden Schritte:

Bestimmen der Menge oder der Fraktion des Proteins, das denaturiert ist, und der Fraktion des denaturierten Proteins, das aggregiert ist, in den mehreren mindestens zweiten Lösungen; und
Vergleichen der Menge oder der Fraktion des Proteins, das aggregiert ist, oder der Menge oder der Fraktion des Proteins, das denaturiert ist, in den mehreren ersten Lösungen mit der Menge oder der Fraktion des Proteins, das aggregiert ist, oder der Menge oder der Fraktion des Proteins, das denaturiert ist, in den mehreren mindestens zweiten Lösungen.

**Revendications**

**1.** Procédé pour la détermination de la hauteur ou proportion d'agrégation de protéine dans une solution sur la base de la concentration en protéine de ladite solution, comprenant :

l'obtention d'une pluralité de premières solutions comprenant des concentrations croissantes d'une protéine ;
la mesure d'une propriété observable pour chaque solution dans ladite pluralité de premières solutions ;
la détermination de ΔG pour la stabilité conformationnelle de ladite protéine à chaque concentration dans ladite pluralité de premières solutions sur la base de ladite propriété observable ;
l'établissement d'une corrélation entre ΔG et chaque concentration de ladite protéine ; et
l'utilisation de ladite corrélation pour déterminer la quantité ou proportion de ladite protéine qui est agrégée.

**2.** Procédé selon la revendication 1, dans lequel chaque solution dans ladite pluralité de premières solutions ne diffère sur aucun paramètre ou diffère sur pas plus de trois paramètres choisis dans le groupe constitué par la composition du tampon, la force du tampon, le pH, la force ionique, la composition de l'excipient, la concentration de l'excipient, la composition du dénaturant chimique et la concentration du dénaturant chimique.

**3.** Procédé selon la revendication 1, comprenant en outre :

l'obtention d'une pluralité au moins de secondes solutions comprenant des concentrations croissantes de ladite protéine ;
la mesure d'une propriété observable pour chaque solution dans ladite pluralité au moins de secondes solutions ;
la détermination de ΔG pour la stabilité conformationnelle de ladite protéine à chaque concentration dans ladite pluralité au moins de secondes solutions sur la base de ladite propriété observable ;
l'établissement d'une corrélation entre ΔG et chaque concentration de ladite protéine dans ladite pluralité au moins de secondes solutions ; et
l'utilisation de ladite corrélation pour déterminer la quantité ou proportion de ladite protéine qui est agrégée dans ladite pluralité au moins de secondes solutions ;
dans lequel ladite pluralité au moins de secondes solutions diffère de ladite pluralité de premières solutions sur un ou plusieurs paramètres choisis dans le groupe constitué par la composition du tampon, la force du tampon, le pH, la force ionique, la composition de l'excipient, la concentration de l'excipient, la composition du dénaturant chimique et la concentration du dénaturant chimique.

**4.** Procédé selon la revendication 3, dans lequel chaque solution dans ladite pluralité au moins de secondes solutions ne diffère sur aucun paramètre ou diffère sur pas plus de trois paramètres choisis dans le groupe constitué par la composition du tampon, la force du tampon, le pH, la force ionique, la composition de l'excipient, la concentration de l'excipient, la composition du dénaturant chimique et la concentration du dénaturant chimique.

**5.** Procédé selon la revendication 3, dans lequel ladite pluralité au moins de secondes solutions comprend des solutions ayant les mêmes concentrations de ladite protéine que ladite pluralité de premières solutions.

**6.** Procédé selon la revendication 3, comprenant en outre l'établissement, pour chaque solution de la pluralité des premières solutions et chaque solution de la pluralité au moins de secondes solutions, d'une corrélation entre ΔG et un paramètre choisi dans le groupe constitué par la composition du tampon, la force du tampon, le pH, la force ionique, la composition de l'excipient et la concentration de l'excipient.

**7.** Procédé selon la revendication 3, comprenant en outre l'établissement d'une corrélation entre la quantité ou proportion de ladite protéine qui est agrégée et un paramètre choisi dans le groupe constitué par la composition du tampon, la force du tampon, le pH, la force ionique, la composition de l'excipient et la concentration de l'excipient.

**8.** Procédé selon la revendication 3, comprenant en outre la comparaison de la quantité ou proportion de ladite protéine qui est agrégée dans ladite pluralité de premières solutions avec la quantité ou proportion de ladite protéine qui est agrégée dans ladite pluralité au moins de secondes solutions.

**9.** Procédé selon la revendication 1, dans lequel 1 ng/ml ou moins de protéine dénaturée ou 1 ng/ml ou moins de protéine agrégée est détectée ou déterminée.

**10.** Procédé selon la revendication 1, dans lequel la quantité ou proportion de la protéine qui est agrégée est déterminée en moins de 24 heures.

**11.** Procédé selon la revendication 1, dans lequel chaque solution dans ladite pluralité de premières solutions comprend 0,1 pg/ml à 2 mg/ml de ladite protéine.

**12.** Procédé selon la revendication 3, dans lequel chaque solution dans ladite pluralité au moins de secondes solutions comprend 0,1 μg/ml à 2 mg/ml de ladite protéine.

**13.** Procédé selon la revendication 1, dans lequel ladite propriété observable est la fluorescence.

**14.** Procédé selon la revendication 3, dans lequel ladite pluralité au moins de secondes solutions diffère de ladite pluralité de premières solutions sur au moins la concentration du dénaturant chimique.

**15.** Procédé selon la revendication 1, comprenant en outre une étape d'identification d'une concentration en protéine qui augmente au maximum ΔG et réduit au minimum la dépendance de ΔG à la concentration en protéine.

**16.** Procédé selon la revendication 1, comprenant en outre une étape d'utilisation de ladite corrélation pour déterminer la quantité ou proportion de ladite protéine qui est dénaturée et la proportion de ladite protéine dénaturée qui est agrégée dans ladite pluralité de premières solutions.

**17.** Procédé selon la revendication 16, comprenant en outre les étapes de
détermination de la quantité ou proportion de ladite protéine qui est dénaturée et de la proportion de ladite protéine dénaturée qui est agrégée dans ladite pluralité au moins de secondes solutions ; et
comparaison de la quantité ou proportion de ladite protéine qui est agrégée ou de la quantité ou proportion de ladite protéine qui est dénaturée dans ladite pluralité de premières solutions avec la quantité ou proportion de ladite protéine qui est agrégée ou la quantité ou proportion de ladite protéine qui est dénaturée dans ladite pluralité au moins de secondes solutions.

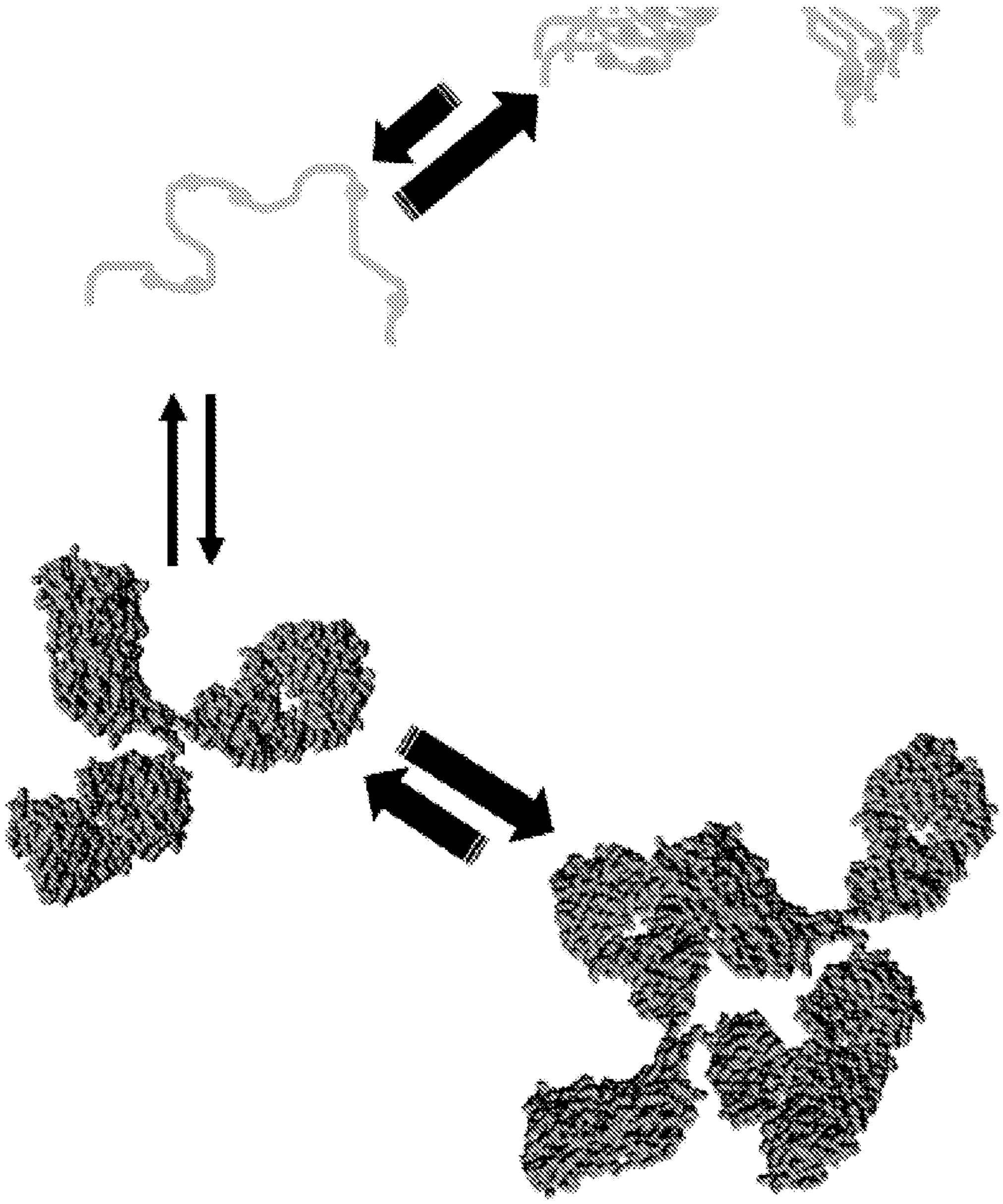

Figure 1

Figure 2

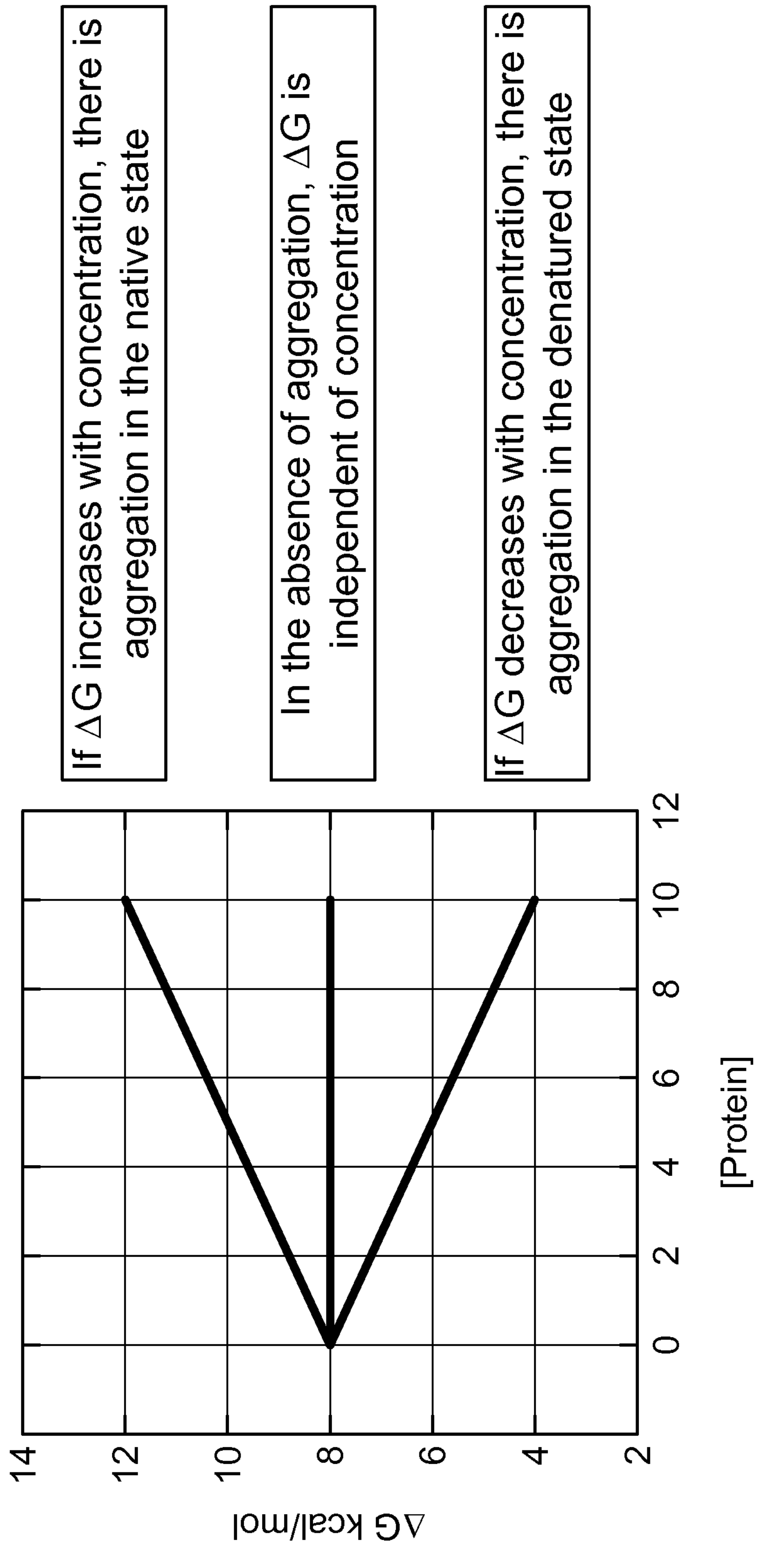
If ΔG increases with concentration, there is aggregation in the native state
In the absence of aggregation, ΔG is independent of concentration
If ΔG decreases with concentration, there is aggregation in the denatured state
ΔG kcal/mol
14
12
10
8
6
4
2
0
2
4
6
8
10
12
[Protein]

Figure 3

$$\Delta G_{measured} = f(T, pH, Salt, Ligand, Excipient, Concentration, Time)$$

Figure 4

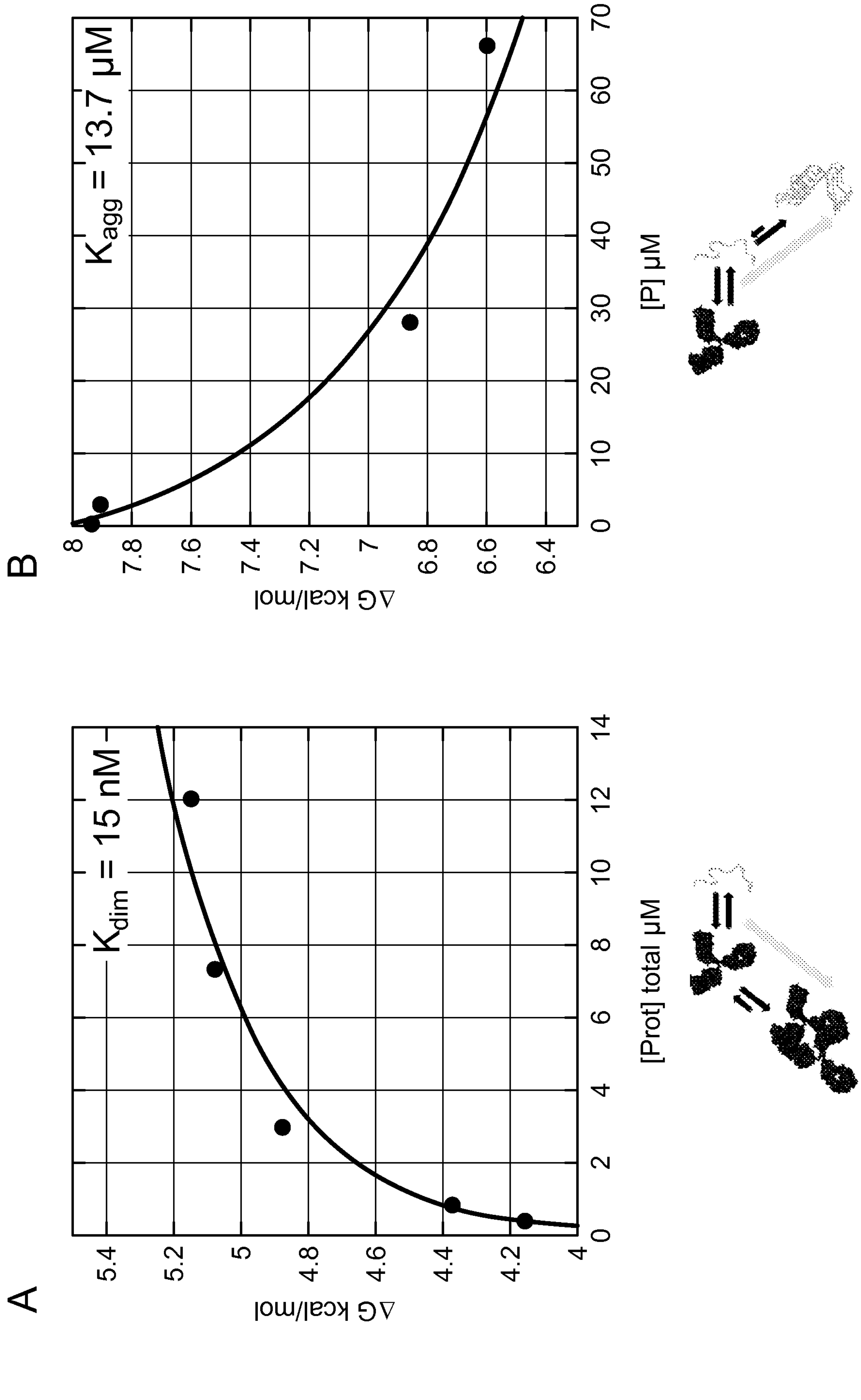
A
$K_{dim}$ = 15 nM
ΔG kcal/mol
[Prot] total μM
B
$K_{agg}$ = 13.7 μM
ΔG kcal/mol
[P] μM

Figure 5

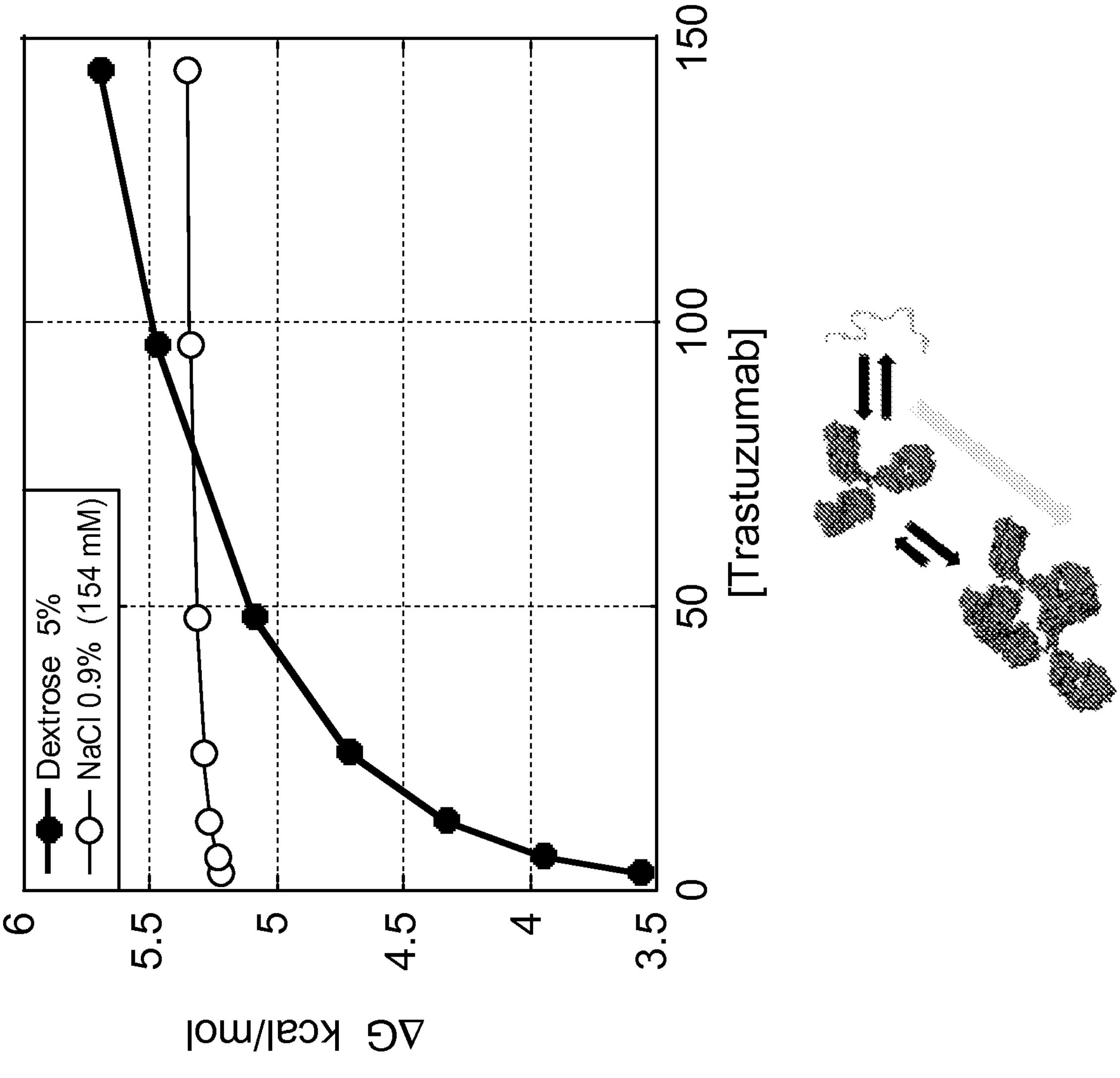
ΔG kcal/mol
6
5.5
5
4.5
4
3.5
0
50
100
150
[Trastuzumab]
Dextrose 5%
NaCl 0.9% (154 mM)

Figure 6

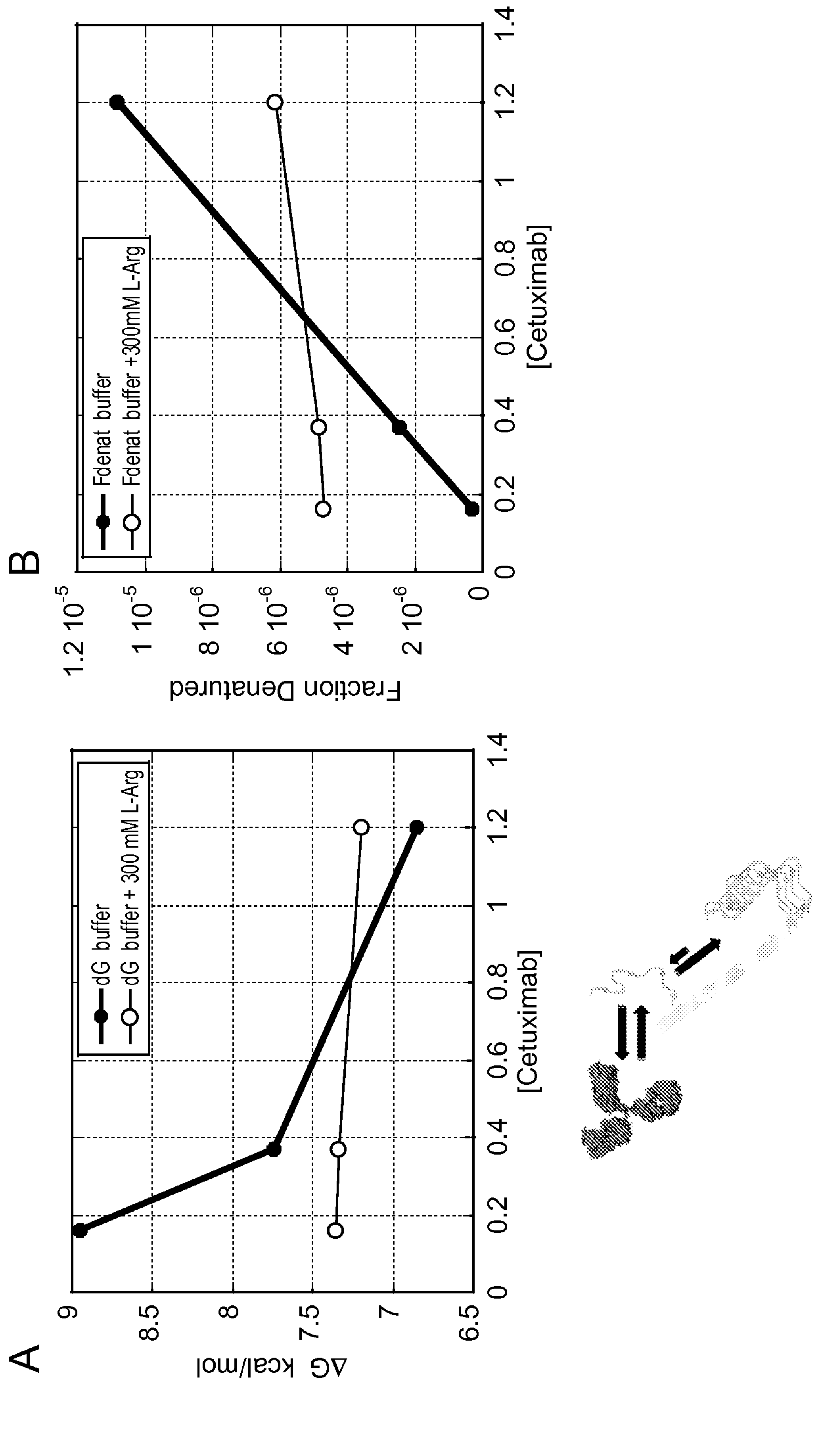
A
ΔG kcal/mol
9
8.5
8
7.5
7
6.5
0
0.2
0.4
0.6
0.8
1
1.2
1.4
[Cetuximab]
dG buffer
dG buffer + 300 mM L-Arg
B
Fraction Denatured
1.2 10-5
1 10-5
8 10-6
6 10-6
4 10-6
2 10-6
0
Fdenat buffer
Fdenat buffer +300mM L-Arg

Figure 6 (Continued)

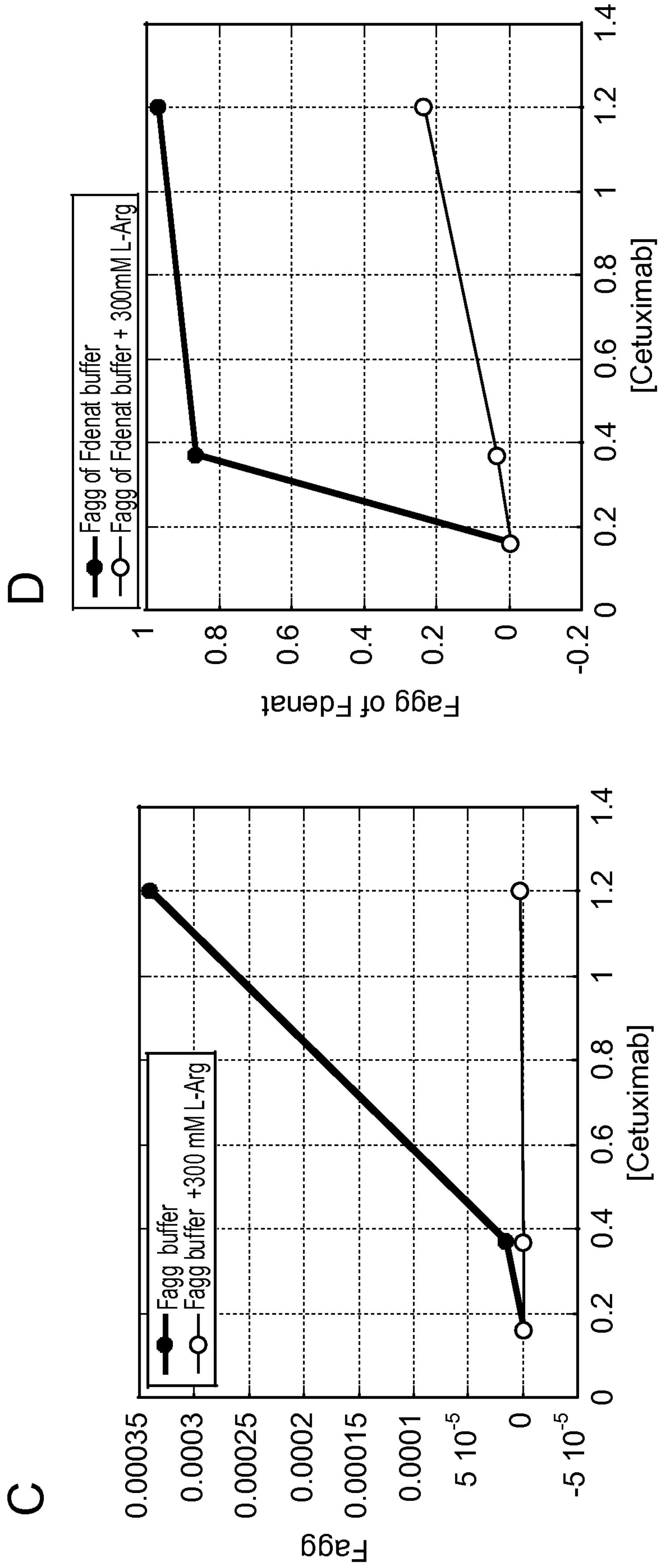

Figure 7

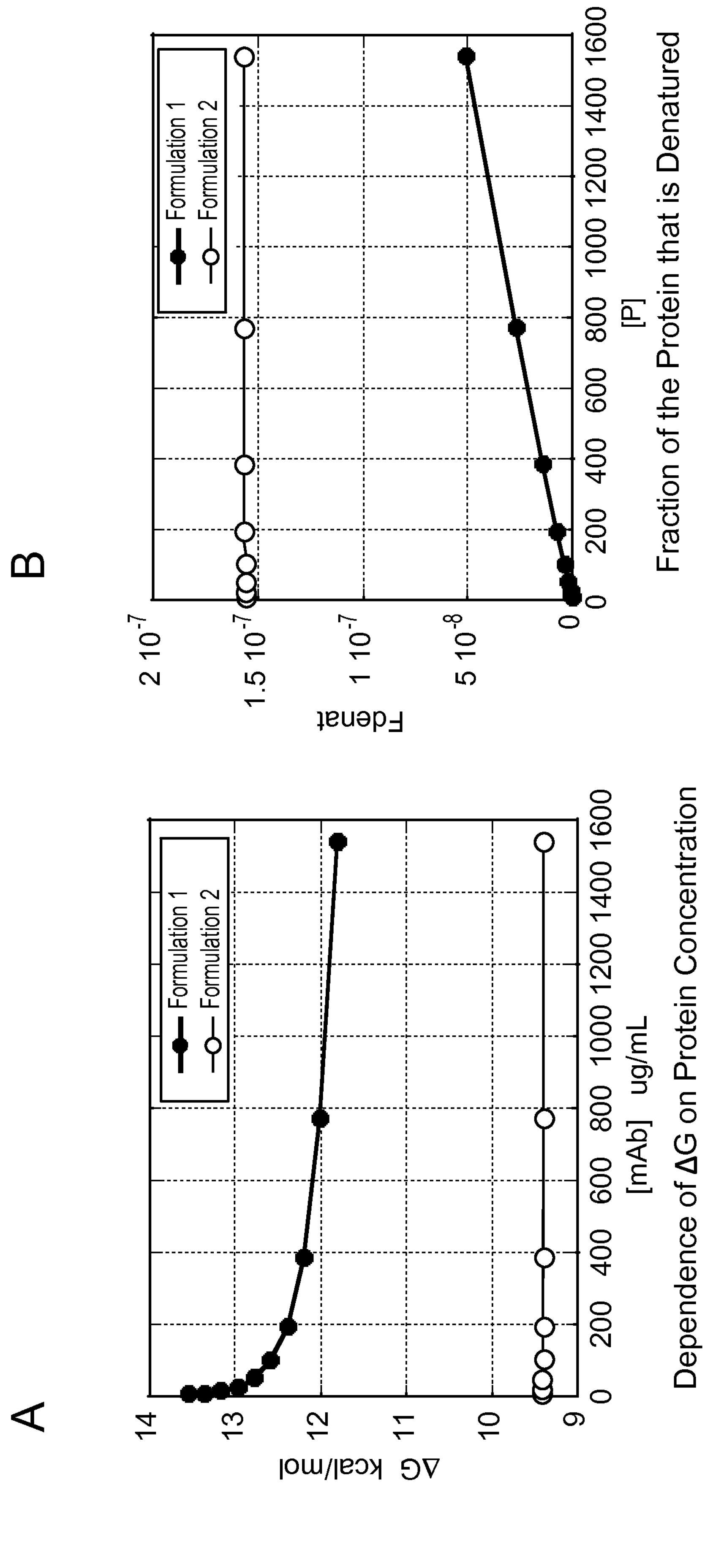
A
ΔG kcal/mol
14
13
12
11
10
9
0
200
400
600
800
1000
1200
1400
1600
[mAb] ug/mL
Formulation 1
Formulation 2
Dependence of ΔG on Protein Concentration
B
Fdenat
2 10-7
1.5 10-7
1 10-7
5 10-8
0
[P]
Formulation 1
Formulation 2
Fraction of the Protein that is Denatured

Figure 7 (Continued)

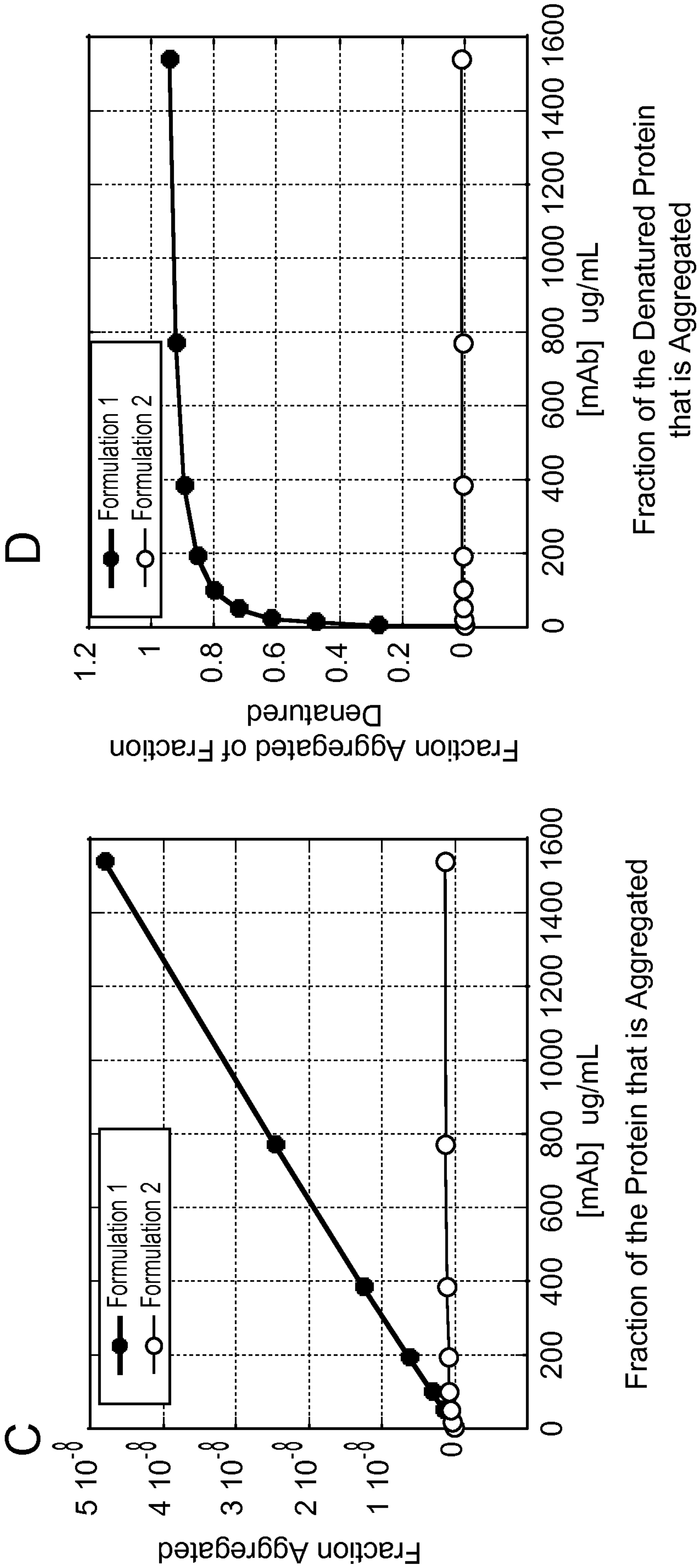

Figure 8

mAb-A pH 6.25

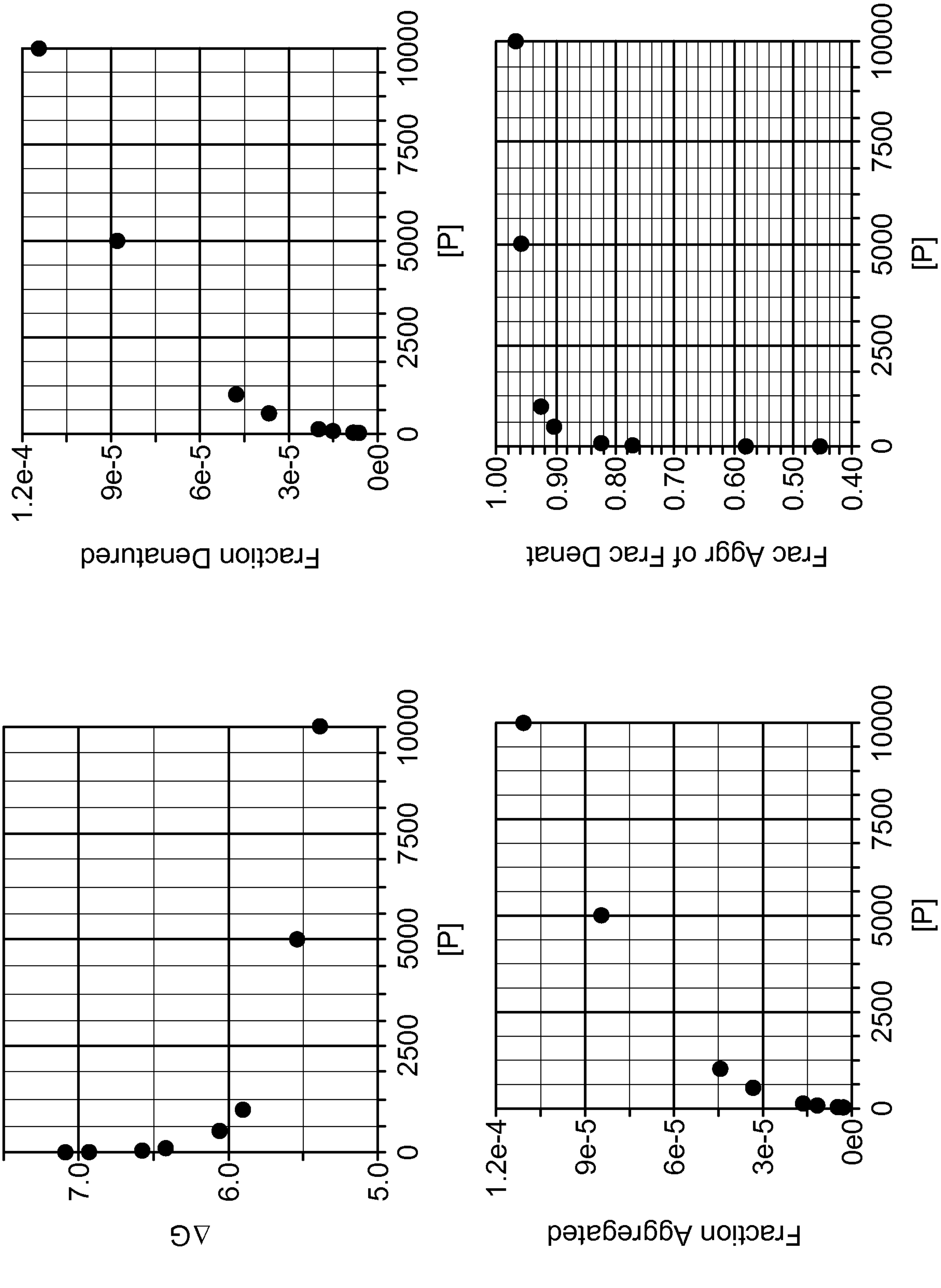

Figure 9

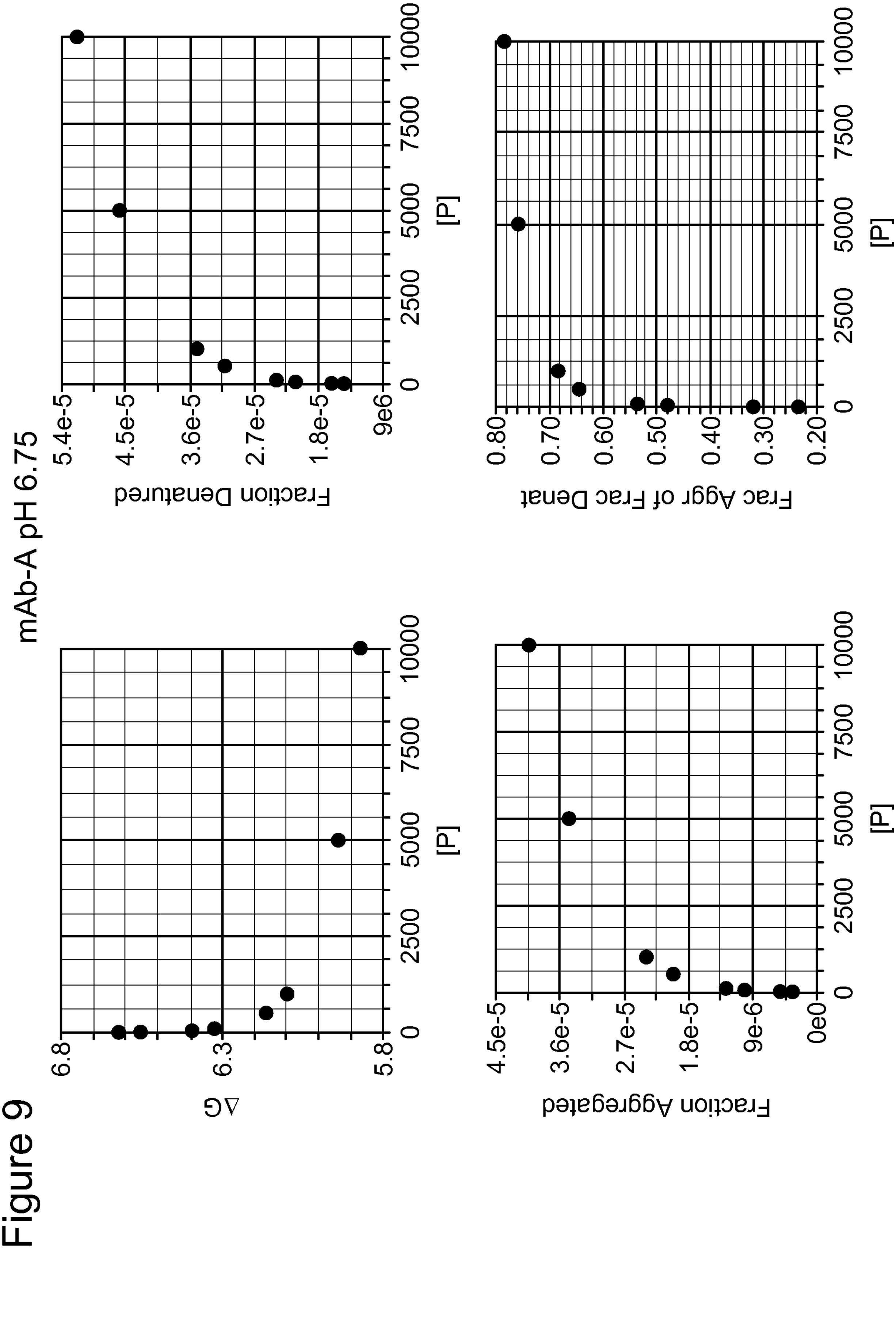
mAb-A pH 6.75
ΔG
6.8
6.3
5.8
Fraction Denatured
5.4e-5
4.5e-5
3.6e-5
2.7e-5
1.8e-5
9e6
Fraction Aggregated
4.5e-5
3.6e-5
2.7e-5
1.8e-5
9e-6
0e0
Frac Aggr of Frac Denat
0.80
0.70
0.60
0.50
0.40
0.30
0.20
0
2500
5000
7500
10000
[P]

# Figure 10

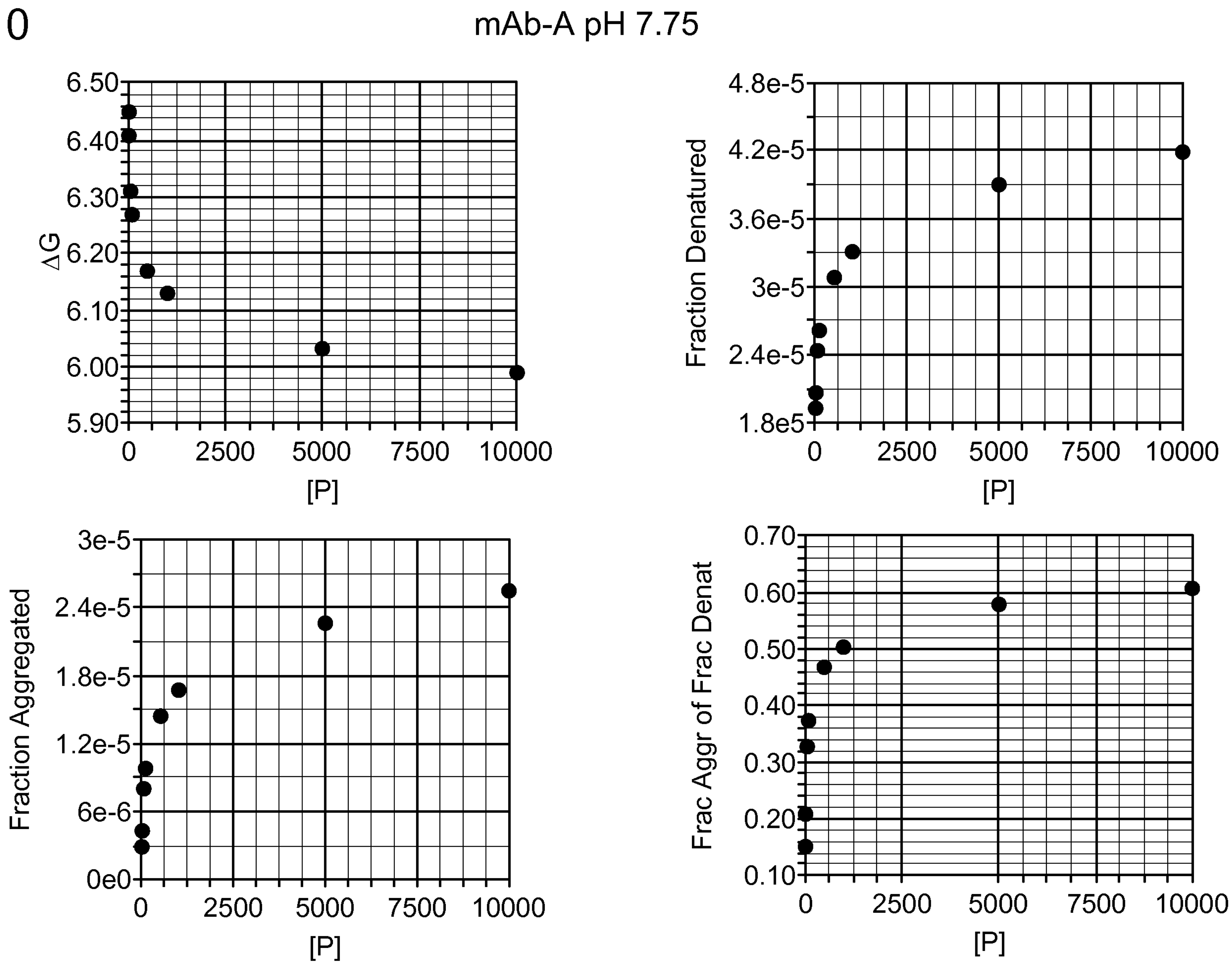
mAb-A pH 7.75
ΔG
6.50
6.40
6.30
6.20
6.10
6.00
5.90
Fraction Denatured
4.8e-5
4.2e-5
3.6e-5
3e-5
2.4e-5
1.8e5
Fraction Aggregated
3e-5
2.4e-5
1.8e-5
1.2e-5
6e-6
0e0
Frac Aggr of Frac Denat
0.70
0.60
0.50
0.40
0.30
0.20
0.10
0
2500
5000
7500
10000
[P]

Figure 11

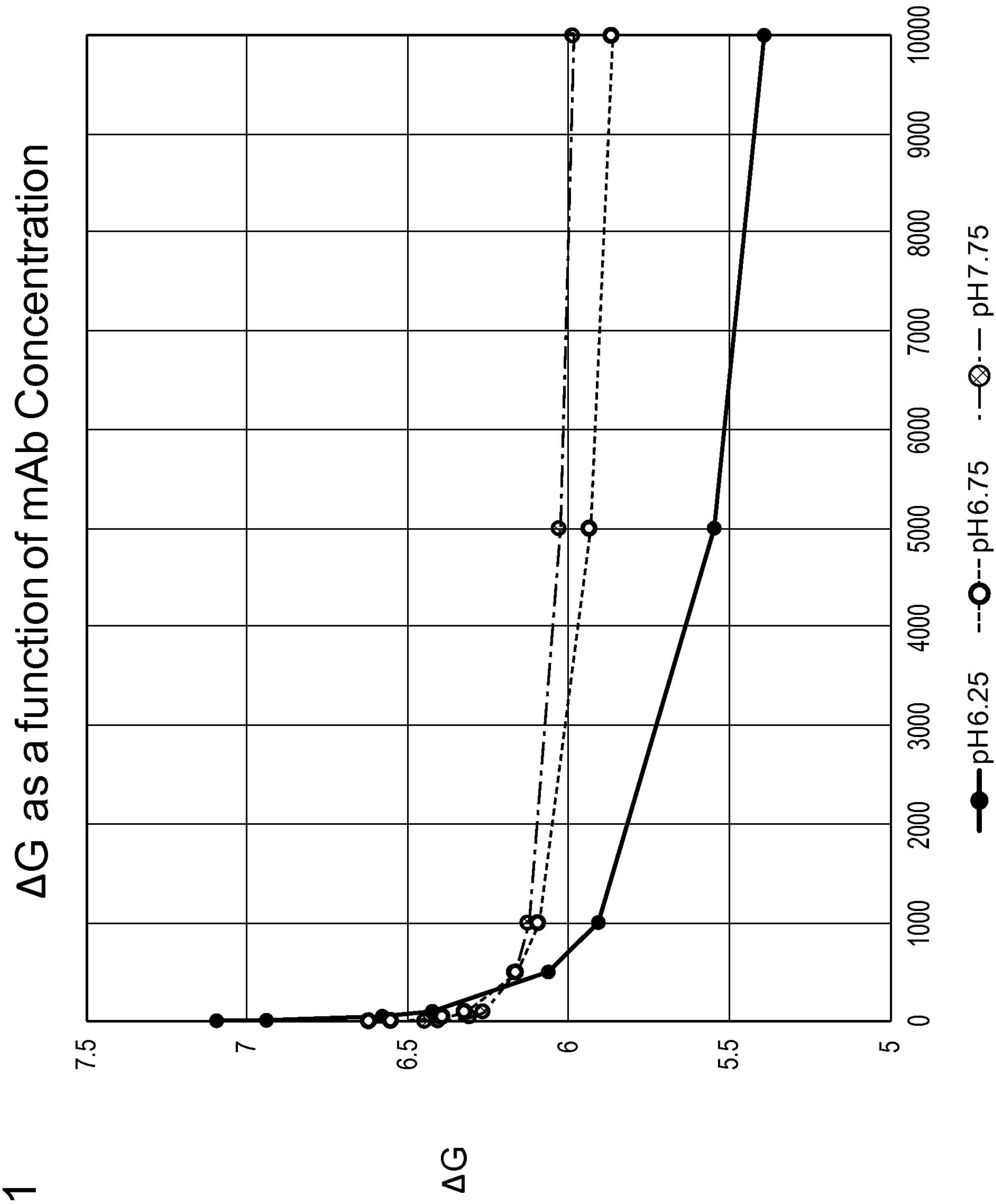
ΔG as a function of mAb Concentration
ΔG
7.5
7
6.5
6
5.5
5
0
1000
2000
3000
4000
5000
6000
7000
8000
9000
10000
pH6.25
pH6.75
pH7.75

Figure 12

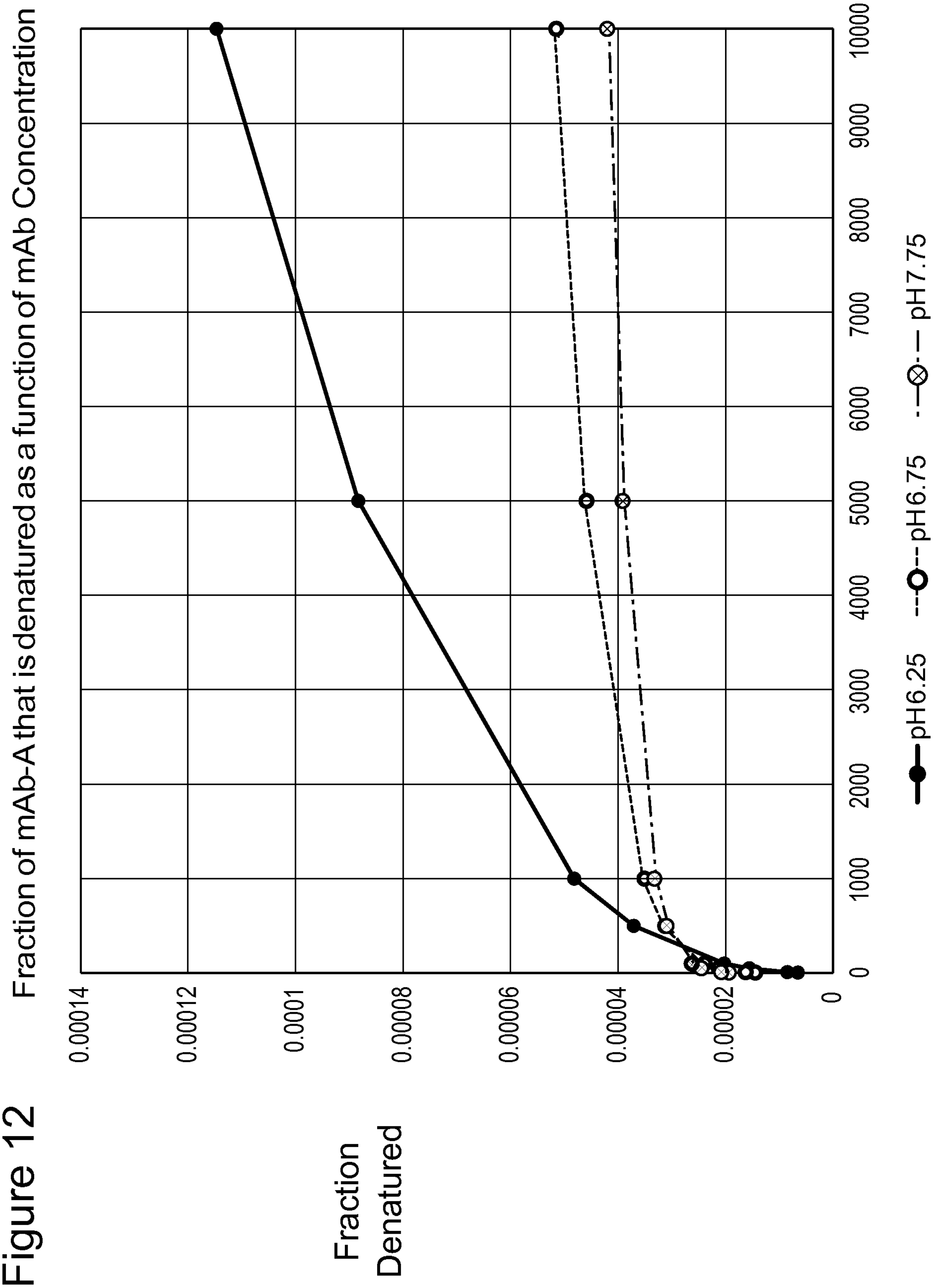
Fraction of mAb-A that is denatured as a function of mAb Concentration
Fraction Denatured
0.00014
0.00012
0.0001
0.00008
0.00006
0.00004
0.00002
0
0
1000
2000
3000
4000
5000
6000
7000
8000
9000
10000
pH6.25
pH6.75
pH7.75

Figure 13

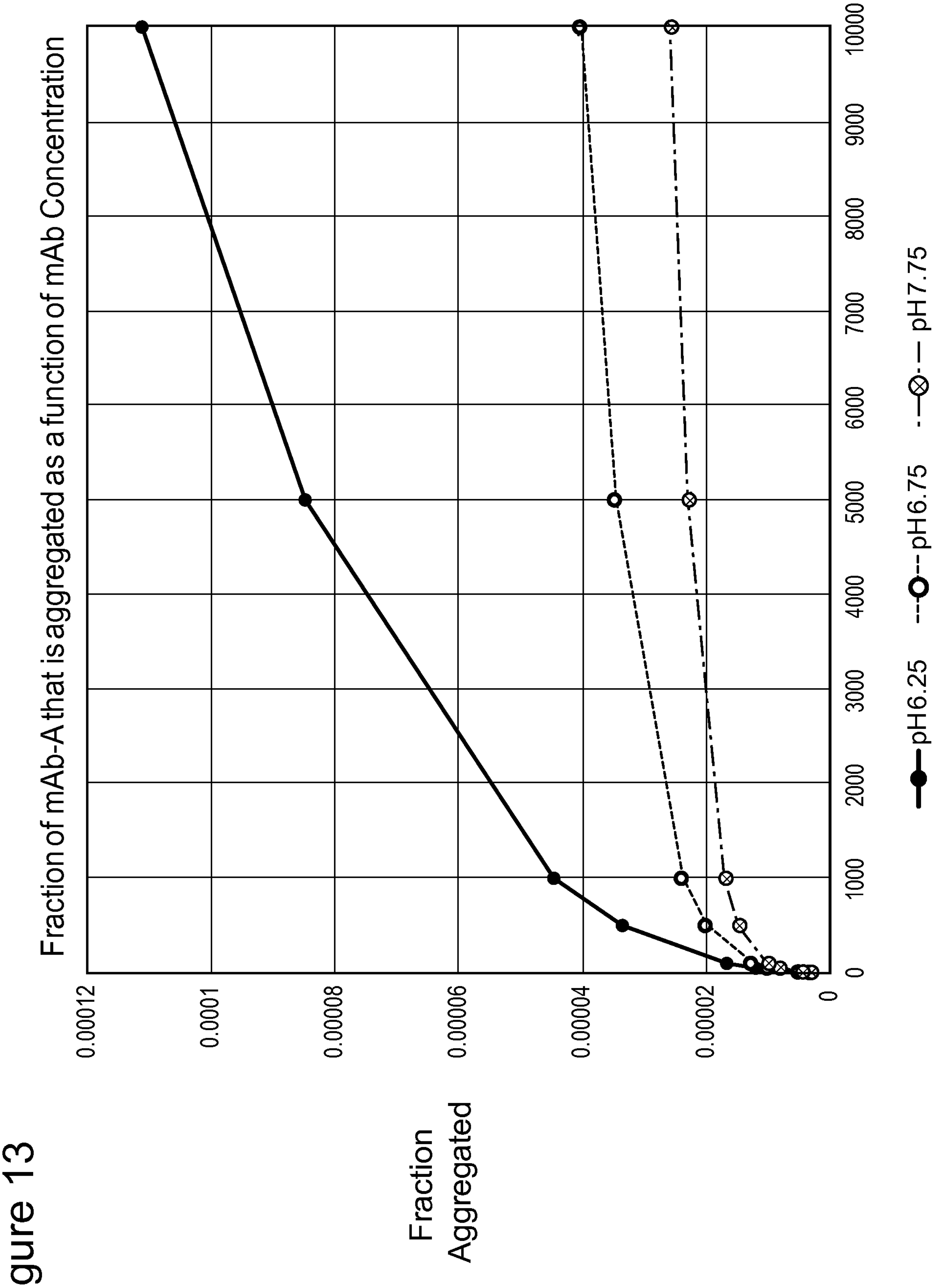
Fraction of mAb-A that is aggregated as a function of mAb Concentration
Fraction Aggregated
0.00012
0.0001
0.00008
0.00006
0.00004
0.00002
0
0
1000
2000
3000
4000
5000
6000
7000
8000
9000
10000
pH6.25
pH6.75
pH7.75

Figure 14

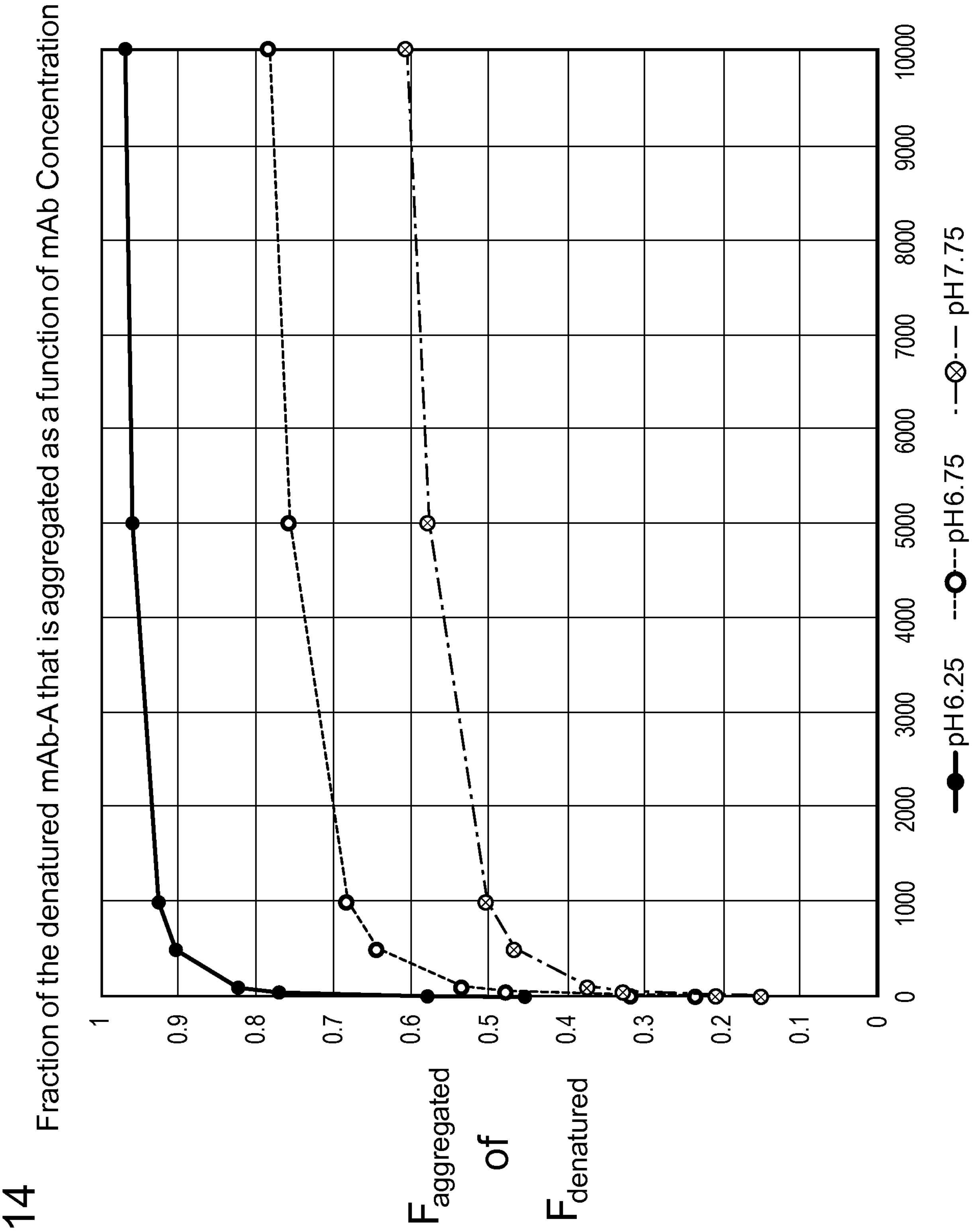
Fraction of the denatured mAb-A that is aggregated as a function of mAb Concentration
$F_{aggregated}$ of $F_{denatured}$
1
0.9
0.8
0.7
0.6
0.5
0.4
0.3
0.2
0.1
0
1000
2000
3000
4000
5000
6000
7000
8000
9000
10000
pH6.25
pH6.75
pH7.75

Figure 15

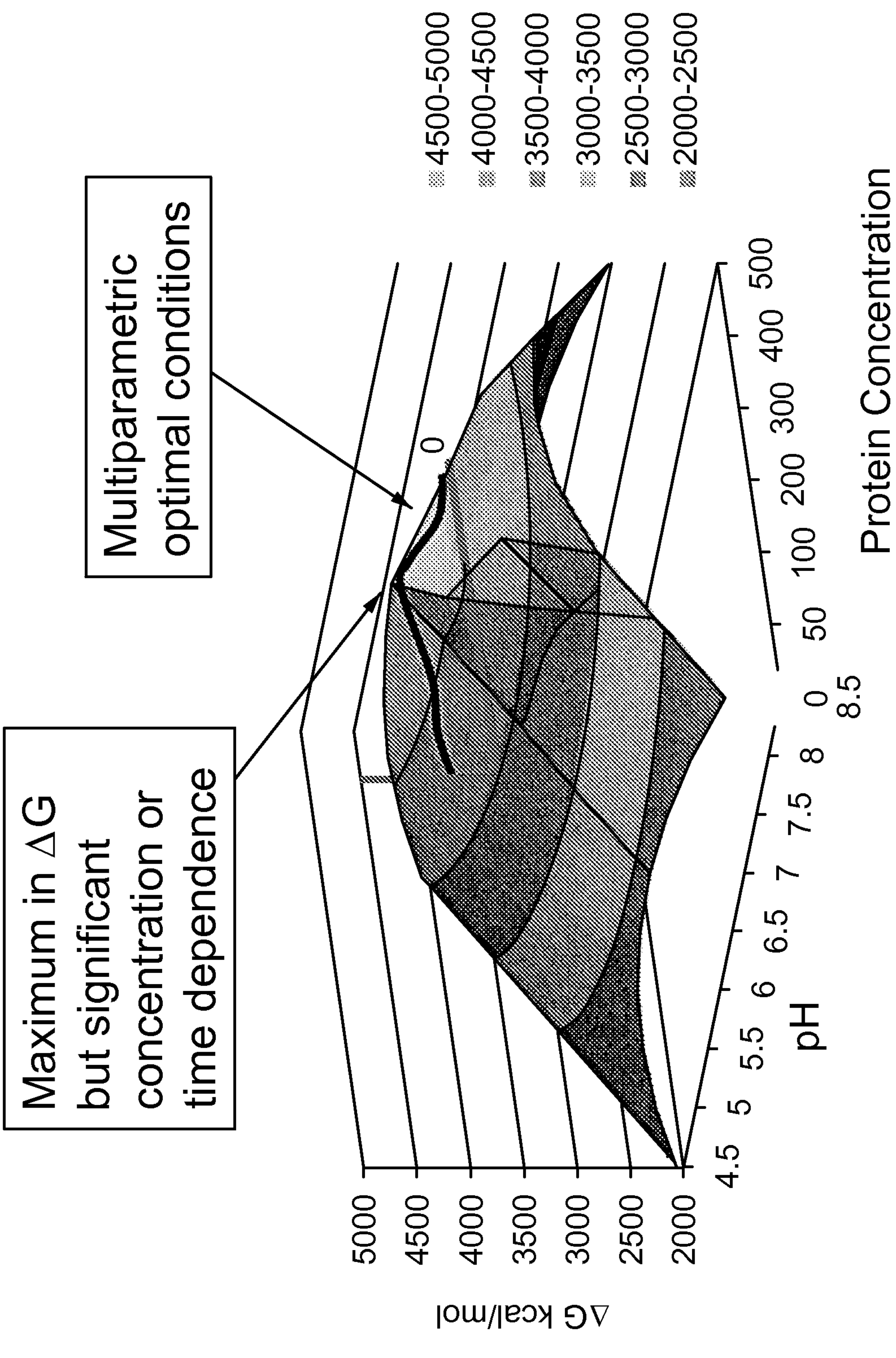
Maximum in ΔG but significant concentration or time dependence
Multiparametric optimal conditions
ΔG kcal/mol
5000
4500
4000
3500
3000
2500
2000
pH
4.5
5
5.5
6
6.5
7
7.5
8
8.5
Protein Concentration
0
50
100
200
300
400
500
4500-5000
4000-4500
3500-4000
3000-3500
2500-3000
2000-2500

Figure 16

| | | | | | | | |
|---|---|---|---|---|---|---|---|
| First Plurality of Solutions | 0.1 µg protein<br>pH 6.25<br>0 M urea | 0.2 µg protein<br>pH 6.25<br>0 M urea | 0.5 µg protein<br>pH 6.25<br>0 M urea | 1 µg protein<br>pH 6.25<br>0 M urea | 10 µg protein<br>pH 6.25<br>0 M urea | 100 µg protein<br>pH 6.25<br>0 M urea | 1000 µg protein<br>pH 6.25<br>0 M urea |
| Second Plurality of Solutions | 0.1 µg protein<br>pH 6.25<br>3 M urea | 0.2 µg protein<br>pH 6.25<br>3 M urea | 0.5 µg protein<br>pH 6.25<br>3 M urea | 1 µg protein<br>pH 6.25<br>3 M urea | 10 µg protein<br>pH 6.25<br>3 M urea | 100 µg protein<br>pH 6.25<br>3 M urea | 1000 µg protein<br>pH 6.25<br>3 M urea |
| Third Plurality of Solutions | 0.1 µg protein<br>pH 6.25<br>4 M urea | 0.2 µg protein<br>pH 6.25<br>4 M urea | 0.5 µg protein<br>pH 6.25<br>4 M urea | 1 µg protein<br>pH 6.25<br>4 M urea | 10 µg protein<br>pH 6.25<br>4 M urea | 100 µg protein<br>pH 6.25<br>4 M urea | 1000 µg protein<br>pH 6.25<br>4 M urea |
| Fourth Plurality of Solutions | 0.1 µg protein<br>pH 6.25<br>5 M urea | 0.2 µg protein<br>pH 6.25<br>5 M urea | 0.5 µg protein<br>pH 6.25<br>5 M urea | 1 µg protein<br>pH 6.25<br>5 M urea | 10 µg protein<br>pH 6.25<br>5 M urea | 100 µg protein<br>pH 6.25<br>5 M urea | 1000 µg protein<br>pH 6.25<br>5 M urea |
| Fifth Plurality of Solutions | 0.1 µg protein<br>pH 6.75<br>5 M urea | 0.2 µg protein<br>pH 6.75<br>5 M urea | 0.5 µg protein<br>pH 6.75<br>5 M urea | 1 µg protein<br>pH 6.75<br>5 M urea | 10 µg protein<br>pH 6.75<br>5 M urea | 100 µg protein<br>pH 6.75<br>5 M urea | 1000 µg protein<br>pH 6.75<br>5 M urea |
| Sixth Plurality of Solutions | 0.1 µg protein<br>pH 7.75<br>5 M urea | 0.2 µg protein<br>pH 7.75<br>5 M urea | 0.5 µg protein<br>pH 7.75<br>5 M urea | 1 µg protein<br>pH 7.75<br>5 M urea | 10 µg protein<br>pH 7.75<br>5 M urea | 100 µg protein<br>pH 7.75<br>5 M urea | 1000 µg protein<br>pH 7.75<br>5 M urea |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62026984 A
- US 62034504 A
- US 8859295 B
- US 8609040 B
- US 9029163 B
- US 20120045846 A

### Non-patent literature cited in the description


- **U.B. ERICSSON ; B.M. HALLBERG ; G.T. DETITTA ; N. DEKKER ; P. NORDLUND.** Thermofuor-based high-throughput stability optimization of proteins for structural studies. *Anal. Biochem,* 2006, vol. 357, 289-298
- **M. VEDADI ; F.H. NIESEN ; A. ALLALI-HASSANI ; O.Y. FEDOROV ; P.J. FINERTY JR ; G.A. WASNEY ; R. YEUNG ; C. ARROWSMITH ; L.J. BALL ; H. BERGLUND.** Chemical screening methods to identify ligands that promote protein stability, protein crystallization and structure determination. *Proc. Natl. Acad. Sci. (USA),* 2006, vol. 103, 15835-15840
- **M.A.H. CAPELLE ; R. GURNY ; T. ARVINTE.** High throughput screening of protein formulation stability: Practical considerations. *Eurpean J. Pharmaceutics and Biopharmaceutics,* 2007, vol. 65, 131-148
- **G.A. SENISTERRA ; P.J. FINERTY JR.** High throughput methods of assessing protein stability and aggregation. *Mol. BioSyst.,* 2009, vol. 5, 217-223
- **E.Y. CHI ; S. KRISHNAN ; T.W. RANDOLPH ; J.F. CARPENTER.** Physical Stability of Proteins in Aqueous Solution: Mechanism and Driving Forces in Nonnative Protein Aggregation. *Pharmaceutical Research,* 2003, vol. 20, 1325-1336
- **O. BOUDKER ; M.J. TODD ; E. FREIRE.** The structural stability of the co-chaperonin GroES. *Journal of molecular biology,* 1997, vol. 272, 770-779
- **M.J. TODD ; N. SEMO ; E. FREIRE.** The structural stability of the HIV-1 protease. *Journal of molecular biology,* 1998, vol. 283, 475-488
- **R.F. GREENE, JR. ; C.N. PACE.** Urea and guanidine hydrochloride denaturation of ribonuclease, lysozyme, alpha-chymotrypsin, and beta-lactoglobulin. *J. Biol. Chem.,* 1974, vol. 249, 5388-5393
- **C.N. PACE ; D.V. LAURENTS ; R.E. ERICKSON.** Urea denaturation of barnase: pH dependence and characterization of the unfolded state. *Biochemistry,* 1992, vol. 31, 2728-2734
- **M.M. SANTORO ; D.W. BOLEN.** Unfolding free energy changes determined by the linear extrapolation method. 1. Unfolding of phenylmethanesulfonyl.alpha.chymotrypsin using different denaturants. *Biochemistry,* 1988, vol. 27, 8063-8068
- **D.W. BOLEN ; M.M. SANTORO.** Unfolding free energy changes determined by the linear extrapolation method. 2. Incorporation of dGn-u values in a thermodynamic cycle. *Biochemistry,* 1988, vol. 27, 8069-8074
- **J.K. MYERS ; C.N. PACE ; J.M. SCHOLTZ.** Denaturant m values and heat capacity changes: Relation to changes in accessible surface area of protein unfolding. *Prot. Science,* 1995, vol. 4, 2138-2148
- **C.N. PACE.** Determination and analysis of urea and guanidine hydrochloride denaturation curves. *Methods Enzymol,* 1986, vol. 131, 266-280
- **R.M. IONESCU ; J. VLASAK ; C. PRICE ; M. KIRCHMEIER.** Contribution of variable domains to the stability of humanized IgG1 monoclonal antibodies. *Journal of Pharmaceutical Sciences,* 2008, vol. 97, 1414-1426
- **E. FREIRE ; A. SCHON ; B.M. HUTCHINS ; R.K. BROWN.** Chemical denaturation as a tool in the formulation optimization of biologics. *Drug Discovery Today,* 2013, vol. 18, 1007-1013
- **B. DEMEULE ; C. PALAIS ; G. MACHAIDZE ; R. GURNY ; T. ARVINTE.** *New methods allowing the detection of protein aggregates: A case study on trastuzumab, mAbs,* 2009, vol. 1, 142-150
- **T. ARAKAWA ; D. ELJIMA ; K. TSUMOTO ; N. OBEYAMA ; Y. TANAKA ; Y. KITA ; E.N. TIMASHEFF.** Suppression of protein interactions by arginine: a proposed mechanism of the arginine effects. *Biophys Chem,* 2007, vol. 127, 1-8
- **P. KHEDDO ; M. TRACKA ; J. ARMER ; R.J. DEARMAN ; S. UDDIN ; C.F. VAN DER WALLE ; A.P. GOLOVANOV.** The effect of arginine glutamate on the stability of monoclonal antibodies in solution. *International Journal of Pharmaceutics,* 2014, vol. 473, 126-133

- **M. FUKUDA ; D. KAMEOKA ; T. TORIZAWA ; M. SAITOH ; M. YASUTAKE ; Y. IMAEDA ; A. KIOGA ; A. MIZUTANI.** Thermodynamic and fluorescence analyses to determine mechanisms of IgG1 stabilization and destabilization by arginine. *Pharmaceutical Research,* 2014, vol. 31, 992-1001
- **A. SCHON ; R.K. BROWN ; B. HUTCHINS ; E. FREIRE.** Ligand binding analysis and screening by chemical denaturation shift. *Analytical Biochem,* 2013, vol. 443, 52-57